# EUROPEAN PATENT APPLICATION

(11) **EP 4 772 647 A1**
(43) Date of publication of application: **08.07.2026**
(21) Application number: 23955858.8
(22) Date of filing: 31.10.2023
(51) Int. Cl.: C12P 19/34, C12N 15/113

(54) **PREPARATION METHOD FOR SIRNA FOR INHIBITING PCSK9 GENE EXPRESSION**

(30) Priority: 16.10.2023 CN 202311335115
(71) Applicant: Jilin Asymchem Pharmaceuticals Co., Ltd., Dunhua, Yanbian, Jilin 133710 (CN); Tianjin Asymchem Pharmaceuticals Co., Ltd, Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); LIU, Fang, Tianjin 300457 (CN); MA, Cuiping, Tianjin 300457 (CN); JIA, Xu, Tianjin 300457 (CN); JIANG, Xiangjun, Tianjin 300457 (CN); LI, Shaohe, Tianjin 300457 (CN); HE, Kuifu, Tianjin 300457 (CN); WANG, Zhaoshuai, Tianjin 300457 (CN); CUI, Lixin, Tianjin 300457 (CN); ZHU, Wenxuan, Tianjin 300457 (CN); MA, Congcong, Tianjin 300457 (CN); PANG, Huining, Tianjin 300457 (CN)
(74) Representative: GLAWE DELFS MOLL
(86) International application number: PCT/CN2023/128826
(87) International publication number: WO 2025/081540

(57) **Abstract**

Provided is a method for preparing siRNA for inhibiting PCSK9 gene expression. The siRNA in the method is double-stranded RNA consisting of a sense strand and an antisense strand, which are complementarily paired with each other. The method includes: mixing a sense strand substrate, an antisense strand substrate, and an RNA ligase, utilizing the RNA ligase to catalyze the ligation between the sense strand substrate and the antisense strand substrate with a phosphodiester bond to obtain a sense strand and an antisense strand, thereby obtaining the siRNA, where the sense strand substrate can form the sense strand, and the antisense strand substrate can form the antisense strand; and the sense strand is a nucleic acid as set forth in SEQ ID NO: 45, and the antisense strand is a nucleic acid as set forth in SEQ ID NO: 46. The method solves the problem in the prior art of low purity of such siRNA through preparation, and is applicable in the field of medicine biosynthesis.

## Description

This application is based upon and claims priority to Chinese Patent Application No. 202311335115.1 filed on October 16, 2023, the disclosure of which is hereby incorporated by reference in its entirety.

### Technical Field

The present disclosure relates to the field of medicine biosynthesis, and specifically, to a method for preparing siRNA for inhibiting PCSK9 gene expression.

### Background

Many diseases are caused by abnormal gene expression and mutations. However, these diseases often cannot be cured by traditional small-molecule drugs, making effective gene expression intervention the only viable treatment approach for such diseases. siRNA is a double-stranded RNA oligonucleotide consisting of 19-21 base pairs. In living organisms, gene silencing caused by siRNA-mediated RNA interference (RNAi) represents a crucial manner for regulating gene expression, and may specifically trigger the degradation of target mRNA, thereby inhibiting gene expression. Following the discovery of the RNAi phenomenon, the technology has been rapidly used by researchers in the field of disease treatment, ushering in a tremendous opportunity for the advancement of gene therapy. The technology achieves therapeutic effects by introducing small interfering RNA (siRNA) to specifically inhibit the expression of pathogenic target genes. In 2018, the U.S. FDA approved an siRNA drug for treating peripheral neuropathy caused by hereditary transthyretin amyloidosis, filling a gap in the field of siRNA drug extension and paving the way for future siRNA drug development. Currently, there are five approved siRNA therapies globally. With the development of siRNA drugs for treating hypercholesterolemia, siRNA drugs are becoming a standard mode for drug treatment. The siRNA drugs can typically intervene at the source, have characteristics of addressing both symptoms and root causes, and are fast in target screening, high in treatment efficiency, low in drug toxicity, and strong in specificity, with effects lasting up to six months. This treatment offers the potential for patients to take medication only twice a year, making it a highly promising therapeutic approach.

The synthesis of siRNA primarily relies on the phosphoramidite method, which includes four steps: deprotection, coupling, capping, and oxidation. However, as the chain length increases, the efficiency of chemical reactions, the purity of synthesis, and the yield decline significantly. Oligonucleotides synthesized by the method typically do not exceed 200 nucleotides (nt) in length. Moreover, the synthesis process involves the use of a large number of organic solvents, significantly increasing reaction costs and the cost of three wastes treatment, and also increasing the burden on environmental pollution. Furthermore, the reaction process generates N+1 impurities and N-1 impurities that are difficult to remove. As the chain length increases, the production of impurities rises sharply, increasing the difficulty of after-treatment and significantly reducing the yield. As siRNA becomes increasingly prevalent in treating common diseases, demands for siRNA surge significantly. However, current synthesis methods face substantial challenges in scaling up production due to equipment limitations. Therefore, there is an urgent need to develop a more efficient and green siRNA synthesis method.

### Summary

The present disclosure is mainly intended to provide a method for preparing siRNA for inhibiting PCSK9 gene expression, so as to solve the problem of low purity associated with the preparation of such siRNA in the prior art.

In order to implement the above objective, according to a first aspect of the present disclosure, a method for preparing siRNA for inhibiting PCSK9 gene expression is provided. The siRNA in the method is double-stranded RNA consisting of a sense strand and an antisense strand, which are complementarily paired with each other. The method includes: mixing a sense strand substrate, an antisense strand substrate, and an RNA ligase, utilizing the RNA ligase to catalyze the ligation between the sense strand substrate and the ligation between the antisense strand substrate with phosphodiester bonds to obtain a sense strand and an antisense strand, thereby obtaining the siRNA, where the sense strand substrate can constitute the sense strand, and the antisense strand substrate can constitute the antisense strand; and the sense strand is a nucleic acid as set forth in SEQ ID NO: 45, and the antisense strand is a nucleic acid as set forth in SEQ ID NO: 46.

Further, the 5' end of the sense strand is a hydroxyl group, and the 3' end of the sense strand is an L96 group; the ribonucleotides at positions 1, 2, 3, 4, 5, 6, 8, 10, 12, 13, 14, 15, 16, 17, 18, 19, 20, and 21 of the sense strand have 2' methoxy modifications; the ribonucleotides at positions 7 and 9 have 2' fluoro modifications; the ribonucleotides at positions 1 and 2 are ligated by a phosphorothioate bond, the ribonucleotides at positions 2 and 3 are ligated by a phosphorothioate bond; and the position 11 of sense strand is deoxyribonucleotide, which is a thymine. The 5' end of the antisense strand is a hydroxyl group, and the 3' end of the antisense strand is a hydroxyl group, the ribonucleotides at positions 1, 3, 7, 9, 11, 13, 15, 17, 19, 20, 21, 22 and 23 of the antisense strand have 2' methoxy modifications; the ribonucleotides at positions 2, 4, 5, 6, 8, 10, 12, 14, 16 and 18 have 2' fluoro modifications, the ribonucleotides at positions 1 and 2 are ligated by a phosphorothioate bond, the ribonucleotides at positions 2 and 3 are ligated by a phosphorothioate bond, the ribonucleotides at positions 21 and 22 are ligated by a phosphorothioate bond, and the ribonucleotides at positions 22 and 23 are ligated by a phosphorothioate bond.

Further, the RNA ligase includes one or more of the RNA ligases shown in SEQ ID NO: 4 to SEQ ID NO: 10 or SEQ ID NO: 13 to SEQ ID NO: 40; or an enzyme having greater than 70% identity to any one of the RNA ligases shown in SEQ ID NO: 4 to SEQ ID NO: 10 or SEQ ID NO: 13 to SEQ ID NO: 40 and having an activity of catalyzing the formation of the phosphodiester bond.

Further, the sense strand substrate includes 2 or more substrates, and the antisense strand substrate includes 2 or more substrates. Preferably, the sense strand substrate has a length of 2-19 nt, more preferably 3-17 nt, and further preferably 6-15 nt. Preferably, the antisense strand substrate has a length of 2-21 nt, more preferably 3-19 nt, and further preferably 6-15 nt.

Further, both the sense strand substrate and the antisense strand substrate include 2 substrates, where the sense strand substrate includes a first sense strand substrate and a second sense strand substrate, and the antisense strand substrate includes a first antisense strand substrate and a second antisense strand substrate. The method includes: the first sense strand substrate, the second sense strand substrate, the first antisense strand substrate, and the second antisense strand substrate are mixed, the RNA ligase is utilized to catalyze the ligation between the first sense strand substrate and the second sense strand substrate to form the sense strand, and to catalyze the ligation between the first antisense strand substrate and the second antisense strand substrate to form the antisense strand, and the sense strand and the antisense strand are complementary paired to form the siRNA. Preferably, the sense strand substrate and the antisense strand substrate are annealed, and then mixed with the RNA ligase, so as to obtain the siRNA.

Further, the 3' end of the first sense strand substrate is ligated to the 5' end of the second sense strand substrate under the catalysis of the RNA ligase, forming the sense strand; and the 3' end of the first antisense strand substrate is ligated to the 5' end of the second antisense strand substrate under the catalysis of the RNA ligase, forming the antisense strand. Preferably, the 5' end of the first sense strand substrate is a hydroxyl group, and the 3' end of the first sense strand substrate is a hydroxyl group; and the 5' end of the second sense strand substrate is a phosphate group, and the 3' end of the second sense strand substrate is an L96 group. Preferably, the 5' end of the first antisense strand substrate is a hydroxyl group, and the 3' end of the first antisense strand substrate is a hydroxyl group; and the 5' end of the second antisense strand substrate is a phosphate group, and the 3' end of the second antisense strand substrate is a hydroxyl group.

Further, the first sense strand substrate is a nucleic acid as set forth in SEQ ID NO: 41, and the second sense strand substrate is a nucleic acid as set forth in SEQ ID NO: 42; or the first sense strand substrate is a nucleic acid as set forth in SEQ ID NO: 53, and the second sense strand substrate is a nucleic acid as set forth in SEQ ID NO: 54.

Further, the first antisense strand substrate is a nucleic acid as set forth in SEQ ID NO: 44, and the second antisense strand substrate is a nucleic acid as set forth in SEQ ID NO: 43; or the first antisense strand substrate is a nucleic acid as set forth in SEQ ID NO: 56, and the second antisense strand substrate is a nucleic acid as set forth in SEQ ID NO: 55.

Further, both the sense strand substrate and the antisense strand substrate include 3 substrates; the sense strand substrate includes a first sense strand substrate, a second sense strand substrate, and a third sense strand substrate; and the antisense strand substrate includes a first antisense strand substrate, a second antisense strand substrate, and a third antisense strand substrate. Preferably, the first sense strand substrate is a nucleic acid as set forth in SEQ ID NO: 47; the second sense strand substrate is a nucleic acid as set forth in SEQ ID NO: 48; and the third sense strand substrate is a nucleic acid as set forth in SEQ ID NO: 49. Preferably, the first antisense strand substrate is a nucleic acid as set forth in SEQ ID NO: 52; the second antisense strand substrate is a nucleic acid as set forth in SEQ ID NO: 51; and the third antisense strand substrate is a nucleic acid as set forth in SEQ ID NO: 50.

Further, the concentrations of the sense strand substrate and the antisense strand substrate are both 1 µM to 1M. Preferably, the concentration of the RNA ligase is 0.01 mg/mL to 10 mg/mL. Preferably, the reaction system formed by mixing the sense strand substrate, the antisense strand substrate and the RNA ligase, further comprises ATP, Tris-HCl, MgCl₂, and DTT. Preferably, the reaction temperature of the method is 0-40 °C, more preferably 15-30 °C. Preferably, the reaction time of the method is 0.1-48 h, more preferably 12-24 h.

With the technical solutions of the present disclosure, by utilizing the above method, the ligation between the sense strand substrates is catalyzed by the RNA ligase, so as to form the sense strand of the siRNA, and the ligation between the antisense strand substrates is catalyzed to form the antisense strand of the siRNA, such that the siRNA with a complex structure and a plurality of modifications is prepared by means of biosynthesis. Compared to the preparation of the siRNA using chemical synthesis, the method of the present application is high in purity of products obtained, less in impurity, mild in reaction condition, and suitable for achieving industrial scale-up production.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:
Fig. 1 is a schematic structural diagram of siRNA according to an embodiment of the present disclosure.
Fig. 2 is a schematic structural diagram of an L96 group according to an embodiment of the present disclosure.
Fig. 3 is a diagram of an SDS-PAGE gel electrophoresis result according to Embodiment 2 of the present disclosure.
Fig. 4 is a diagram of liquid chromatography and mass spectrometry detection results according to Embodiment 2 of the present disclosure.
Fig. 5 is a diagram of liquid chromatography and mass spectrometry detection results according to Embodiment 2 of the present disclosure.
Fig. 6 is a diagram of liquid chromatography detection results according to Embodiment 6 of the present disclosure.
Fig. 7 is a diagram of liquid chromatography detection results according to Embodiment 7 of the present disclosure.

### Detailed Description of the Embodiments

It is to be noted that the embodiments in the present application and the features in the embodiments may be combined with one another without conflict. The present disclosure will be described below in detail with reference to the embodiments.

Term explanation:
N-1 impurity: a nucleic acid impurity having a single base deletion compared to a target synthetic sequence.
N+1 impurity: a nucleic acid impurity having a single base addition compared to a target synthetic sequence.

As mentioned in the Background, the preparation of such siRNAs in the prior art is performed by means of chemical synthesis, which not only consumes a large number of organic solvents, not confirming to green chemistry principles, but also yields products with low purity and high impurity levels. In particular, there are N+1 impurities and N-1 impurities difficult to remove, affecting subsequent purification of the products. The inventor in the present application attempts to develop a method for preparing siRNA for inhibiting PCSK9 gene expression. Such siRNA is synthesized using enzyme catalysis, such that a series of protective schemes of the present application are proposed.

In a first typical implementation of the present application, a method for preparing siRNA for inhibiting PCSK9 gene expression is provided. The above siRNA in the method is double-stranded RNA consisting of a sense strand and an antisense strand, which are complementarily paired with each other. The method includes: mixing a sense strand substrate, an antisense strand substrate, and an RNA ligase, utilizing the RNA ligase to catalyze the ligation between the sense strand substrate and the ligation between the antisense strand substrate with phosphodiester bonds to obtain a sense strand and an antisense strand, thereby obtaining the siRNA, where the sense strand substrate can form the sense strand, and the antisense strand substrate can form the antisense strand; and the sense strand is a nucleic acid as set forth in SEQ ID NO: 45, and the antisense strand is a nucleic acid as set forth in SEQ ID NO: 46.

The siRNA of the present application is a double-stranded RNA comprising a sense strand as set forth in SEQ ID NO: 45 and an antisense strand as set forth in SEQ ID NO: 46. In the above method, the sense strand substrate includes two or more nucleotide sequences that can form the sense strand, that is, the plurality of nucleotide sequences of the sense strand substrate can be spliced to form a sequence same as a sense strand sequence. A difference between the sequence and the sense strand lies in that there is a nick between the sense strand substrates, which are not ligated by the phosphodiester bond. Similarly, two or more antisense strand substrates are ligated by the phosphodiester bond using the RNA ligase, so as to obtain the antisense strand of the siRNA.

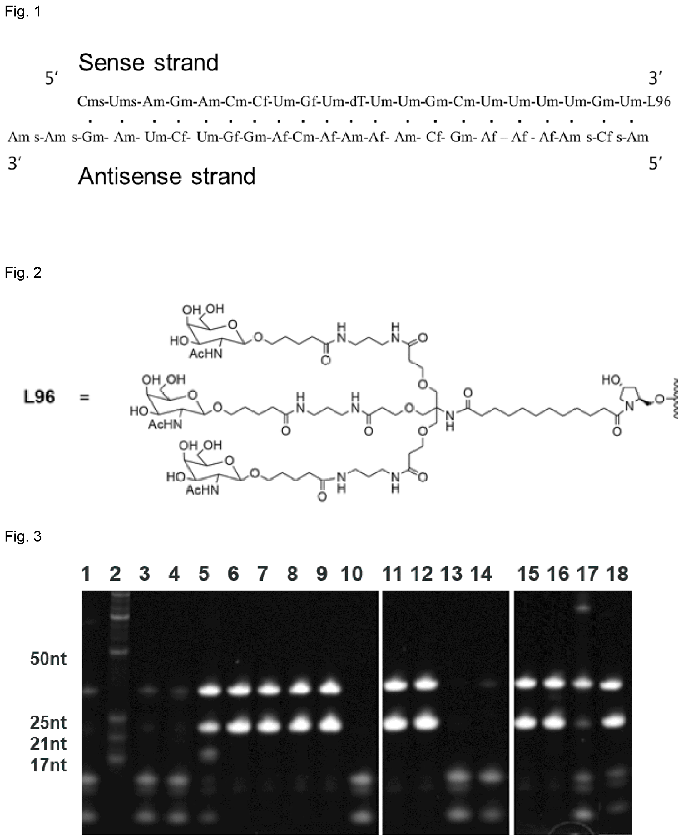

In the above method, the sense strand substrate, the antisense strand substrate, and the RNA ligase are mixed together, and the siRNA is directly prepared through a one-pot method. In the ligation of the one-pot method, complementary base pairing can be performed between the sense strand substrate and the antisense strand substrate, so as to form a double-stranded structure. The RNA ligase identifies the double-stranded structure and then ligates the nicks in the double-stranded structure, thereby obtaining a target product siRNA through preparation. The structure of the target product siRNA is shown in Fig. 1. The siRNA prepared by the above method includes a nucleic acid shown in Fig. 1, and also includes a nucleic acid bound to ions, including, but not limited to, sodium salt forms thereof. The ions bound to the nucleic acid do not affect the proceeding of the above method, the binding of the ions and the nucleic acid can be automatically performed in a reaction system.

In a preferred embodiment, the 5' end of the sense strand is a hydroxyl group, and the 3' end of the sense strand is an L96 group; the ribonucleotides at positions 1, 2, 3, 4, 5, 6, 8, 10, 12, 13, 14, 15, 16, 17, 18, 19, 20, and 21 of the sense strand have 2' methoxy modifications; the ribonucleotides at positions 7 and 9 have 2' fluoro modifications; the ribonucleotides at positions 1 and 2 are ligated by a phosphorothioate bond, the ribonucleotides at positions 2 and 3 are ligated by a phosphorothioate bond; and the position 11 of sense strand is deoxyribonucleotide, which is a thymine. The 5' end of the antisense strand is a hydroxyl group, and the 3' end of the antisense strand is a hydroxyl group, the ribonucleotides at positions 1, 3, 7, 9, 11, 13, 15, 17, 19, 20, 21, 22 and 23 of the antisense strand have 2' methoxy modifications; the ribonucleotides at positions 2, 4, 5, 6, 8, 10, 12, 14, 16 and 18 have 2' fluoro modifications, the ribonucleotides at positions 1 and 2 are ligated by a phosphorothioate bond, the ribonucleotides at positions 2 and 3 are ligated by a phosphorothioate bond, the ribonucleotides at positions 21 and 22 are ligated by a phosphorothioate bond and the ribonucleotides at positions 22 and 23 are ligated by a phosphorothioate bond.

In a preferred embodiment, the RNA ligase includes one or more of the RNA ligases shown in SEQ ID NO: 4 to SEQ ID NO: 10 or SEQ ID NO: 13 to SEQ ID NO: 40; or an enzyme having greater than 70% identity to any one of the RNA ligases shown in SEQ ID NO: 4 to SEQ ID NO: 10 or SEQ ID NO: 13 to SEQ ID NO: 40 and having an activity of catalyzing the formation of the phosphodiester bond.

The above RNA ligases all can identify the double-stranded structure that is formed through complementary pairing of substrates and has nicks, so as to catalyze a phosphate group and a hydroxyl group to form a phosphodiester bond. The RNA ligases have different activities for different substrates. In the above method, the above RNA ligases are obtained by screening a large number of RNA ligases, such that the ligation and preparation of the siRNA with the sense strand being the nucleic acid as set forth in SEQ ID NO: 45, and the antisense strand being the nucleic acid as set forth in SEQ ID NO: 46 are realized.
SEQ ID NO: 1 (E1, Salmonellaenterica): 
MFKKYSSLENHYNSKFIEKLYSLGLTGGEWVAREKIHGTNFSLIIERDKVTCAKRTGPILPAED
FFGYEIILKNYADSIKAVQDIMETSAVVTYQVFGEFAGPGIQKNVDYGDKDFYVFDIIVTTESGDVTY
VDDYMMESFCNTFKFKMAPLLGRGKFEELIKLPNDLDSVVQDYNFTVDHAGLVDANKCVWNAEA
KGEVFTAEGYVLKPCYPSWLPNRNRVAIKCKNSKFSEKKKSDKPIKTKVELSEADNKLMGILACYV
 
TLNRVNNVISKIGEIGPKDFGKVMGLTVQDILEETSREGITLTQADNPSLIKKELVKMVQDVLRPAWI
ELVS.
SEQ ID NO: 2 (E2, YersiniaphagevB_YepM_ZN18): 
MFKKYSSLENHYNSKFIEKLYSLGLTGGEWVAREKIHGTNFSLIIERDKVTCAKRTGPILPAED
FFGYEIILKNYEDSIKAVQDIMETSAVVSYQVFGEFAGPGIQKNVDYGDKDFYVFDIIVTTESGDVTY
VDDYMMESFCNTFKFKMAPLLGRGKFEELIKLPNDLDSVVQDYNFTVDHAGLVDANKCVWKAEA
KGEVFTAEGYVLKPCYPSWLHNGNRVAIKCKNSKFSEKKKSDKPIKAKVELSEADNKLVGILACYV
TLNRVNNVISKIGEIGPKDFGKVMGLTVQDILEETSREGITLTQADNPSLVKKELVKMVQDVLRPAW
IELVS.
SEQ ID NO: 3 (E3, Shigella phage pSs-1): 
MFKKYSSLENHYNSKFIEKLYSLGLTGGEWVAREKIHGTNFSLIIERDKVTCAKRTGPILPAED
FFGYEIILKNYADSIKAVQDIMETSAVVSYQVFGEFAGPGIQKNVDYGDKDFYVFDIIVTTESGDVTY
VDDYMMESFCNTFKFKMAPLLGRGKFEELIKLPNDLDSVVQDYNFTVDHAGLVDANKCVWNAEA
KCEVFTAEGYVLKPCYPSWLPNRNRVAIKCKNSKFSEKKKSDKPIKAKVELSEADNKLVGILACYV
TLNRVNNVISKIGEIGPKDFGKVMGLTVQDILEETSREGITLTQADSPSLVKKELVKMVQDVLRPAW
IELVS .
SEQ ID NO: 4 (E4, Bacteriophage, T4Rnl2): 
MFKKYSSLENHYNSKFIEKLYSLGLTGGEWVAREKIHGTNFSLIIERDKVTCAKRTGPILPAED
FFGYEIILKNYADSIKAVQDIMETSAVVSYQVFGEFAGPGIQKNVDYCDKDFYVFDIIVTTESGDVTY
VDDYMMESFCNTFKFKMAPLLGRGKFEELIKLPNDLDSVVQDYNFTVDHAGLVDANKCVWNAEA
KGEVFTAEGYVLKPCYPSWLRNGNRVAIKCKNSKFSEKKKSDKPIKAKVELSEADNKLVGILACYV
TLNRVNNVISKIGEIGPKDFGKVMGLTVQDILEETSRE
GITLTQADNPSLIKKELVKMVQDVLRPAWIELVS.
SEQ ID NO: 5 (E5, Bacteriophage T7, T7 DNA): 
MNIKTNPFKAVSFVESAIKKALDNAGYLIAEIKYDGVRGNICVDNTANSYWLSRVSKTIPALEHL
NGFDVRWKRLLNDDRCFYKDGFMLDGELMVKGVDFNTGSGLLRTKWTDTKNQEFHEELFVEPIR
KKDKVPFKLHTGHLHIKLYAILPLHIVESGEDCDVMTLLMQEHVKNMLPLLQEYFPEIEWQAAESYE
VYDMVELQQLYEQKRAEGHEGLIVKDPMCIYKRGKKSGWWKMKPENEADGIIQGLVWGTKGLAN
EGKVIGFEVLLESGRLVNATNISRALMDEFTETVKEATLSQWGFFSPYGIGDNDACTINPYDGWAC
QISYMEETPDGSLRHPSFVMFRGTEDNPQEKM.
SEQ ID NO: 6 (E6, Bacteriophage dhaeg): 
MFKKYSSLENHYNSKFIEKLRTNGLTGGEWVAREKIHGTNFSLIIERDAVTCAKRTGPILPAED
FYGYEIVLKNYADSIKSIQDIMETSAAVSYQVFGEFAGTGIQKNVDYGDKDFYVFDIIVTTESGDVTY
VDDYMMESFCKTFKFKMAPLLGRGKFEDLIKLPNDLDSVLPDYNFTVDNVGLAEANAHVWNAEAK
 
GEVFTAEGYVLKPCYPLWLPNGNRVAIKCKNSKFSEKKKTDKPIKVAVVLSQDDLDLLQQFTDYVT
VNRINNVISKIGEVSPKDFGKVMGLTVQDILEEAAREELELTDAENPVEVKKQLIECVKDTLRAVWIE
LVSK.
SEQ ID NO: 7 (E7, Klebsiella phage KP27): 
MFKKYSSLTNHYEGKFINGVIMNGLTGGVWVAREKIHGANFSFITDDGITVTPAKRTDVVKPA
EDFYGCSAVVAKYSPGIRKMWETLKKTGTYDDLVIQVYGEFAGRGVQKDVDYGEKDFYVFDIRVN
GEFLPDNLCSLISRSHGLKMAPLLGYGTFEEIKELPITFESVVNKANSGIGSDNTVYGEFVYPIMDV
EEGNIAEGFVMKPVSPAFMPNGERVAIKCKTTKFTEKKAKKATRFNAPVSLSEKDKNQLDEFVCYL
TENRVKNVLSKLDLASITAKDFGRIMGLTVQDAIEEISRNHGPFLEQFEDPAMAKKLFVTEAQNMIR
PVWGKILNHEF.
SEQ ID NO: 8 (E8, Vibrio phage phi-ST2): 
MSFVKYTSLENSYRQAFVDKCDMLGVKEWVALEKIHGANFSFIVEFKPSTEEVPGEMSVTPA
KRTSTIGANAMGDYDFYGCTSVVEAHIEKMQDISNWLFANDFIKNDETIIVYGELAGKGIQKEVNYG
DKDFWAYDILCPETGEFLDWDVVLKACKFAGVKTTHEIARGTLDELLKIDPLFRSFHTPADVDSEN
VAEGFVVKQLKAEKRLHNGSRAILKVKNEKFKEKKNKQGKTPRAKVVLTEEQEKLHAAFSCYLTE
NRLRNVLSKIGKVEAKQFGMVSGLFVKDAKDEFERDERDEVAIPRDDWDVIKRSLVNVANEILRKN
WLNIVDGTF.
SEQ ID NO: 9 (E9, Klebsiella phage KP15): 
MFKKYSSLTNHYEGKFINGVIMNGLTGGVWVAREKIHGANFSFITDDGITVTPAKRTDVVKPA
EDFYGCSAVVAKYSPGIRKMWETLKKTGTYDDLVIQVYGEFAGRGVQKDVDYGEKDFYVFDIRVN
GEFLPDNLCSLISRSHGLKMAPLLGYGTFEEIKELPITFESVVNKANSGIGSDNTVYGEFVYPIMDV
EEGNIAEGFVMKPVSPAFMPNGERVAIKCKTTKFTEKKAKKATRFNAPVSLSEKDKNQLDEFVCYL
TENRVKNVLSKLDLASITAKDFGRIMGLTVQDAIEEISRNHGPFIEQFEDPAMAKKLFVTEAQNMIR
PVWGKILNHEF.
SEQ ID NO: 10 (E10, Vibrio phage JN02): 
MFKKYSSLENHYNSKFIEKLYTNGLTTGVWVAREKIHGTNFSLIIERDNVTCAKRTGPILPAED
FYGYEIVLKKYDKAIKAVQEVMESISTSVPVSYQVFGEFAGGGIQKGVDYGEKDFYVFDIIINTESDD
TYYMSDYEMQDFCNTFGFKMAPMLGRGTFDSLIMIPNDLDSVLAAYNSTASEDLVEANNCVFDAN
VIGDNTAEGYVLKPCFPKWLSNGTRVAIKCKNSKFSEKKKSDKPVKTQVPLTEIDKNLLDVLACYV
TLNRVNNVISKIGTVTPKDFGKVMGLTVQDILEETSREGIVLTSSDNPNLVKKELVRMVQDVLRPA
WIELVS.
SEQ ID NO: 11 (E11, Bacteriophage RB69): 
MEKLYYNLLSLCKSSSDRKFFYSDDVSPIGKKYRIFSYNFASYSDWLLPDALECRGIMFEMDG
ETPLRIASRPMEKFFNLNENPFTLSIDLNDVKYLMTKEDGSLVSTYLDGNMVRFKGSIKSDQAASA
 
TSILLDINHKDLADRLLELCNDGFTANFEYVAPSNKIVLTYPEKRLILLNIRDNNTGKYIEYDDIYLDPV
FRKYLVDRFEAPEGDWVPGVKSSTNIEGYVAVMKDGSHFKLKTDWYVALHTTRDSISSPEKLFLAI
MNGASDDLKAMYADDEFSFKKVELFEKAYLDFLDRSFYICLDAYDKHKGKDRKTYAIEAQAICKGA
QSPWLFGIIMNLYQGGSKEQMMTALESVFIKNHKNFIPEGY.
SEQ ID NO: 12 (E12, Aeromonas virus Aeh1): 
MIVGRYEKQIRQLHKCLVHLDMIDKEDSIGVFGEYAGIMSYGKWIQTGIEYGPQDFYVFDIIVQ
PLDGDQFMLEDLQVEELCKALGLKTAPLLKVGTFADIMKIPVDLQSVVMEVNAGKPIDIRVGTDNIA
EGYVAKPNVPARFKNGNRVAIKCKNEKWSETGKGRAVQVEVKELSEKDKALVLDITRYITDNRLK
NVLSKMDTPKTSEFGKVLGLMSKDVQKDYERDELDGVSVKEATDEAGRVMRMINTEIGNMIRPN
WVSICDGDW.
SEQ ID NO: 13 (E13, Vibrio phage JS98): 
MFKKYSSLENHYNSKFIEKLRTNGLTGGEWVAREKIHGTNFSLIIERDAVTCAKRTGPILPAED
FYGYEIVLKNYADSIKSVQHLIESINYQSYQIYGELAGPGIQKNVDYGDKDFYVFDIRVTKEDGTESV
LTDTLMEAFCIIHKFKVAPCLATGSFEDLIKLPNDFDSVIPDYNFAVDNAGLTIANSTDFIPKVEGKVF
TAEGFVLKPDIPTWLPNGNRVAIKCKNSKFSEKKKSDKPIKAAVVLSQDDMDLMWQFTDYVTVNRI
NNVISKIGEVSKKDFGKVMGLTVQDILEEAAREELELTDAENPVEVKKQLVECVKDTLRAVWIELVS
.
SEQ ID NO: 14 (E14, Vibrio phage): 
MSFVKYTSLENSYRQAFVDKCDMLGVREWVALEKIHGANFSFIVEFKPNEAQDGAEFTVTPA
KRTSTIGANVMGDYDFYGCTSVVEAHTAKMEAISNWLWARGIINVGETIIVYGELAGKGVQKEVNY
GDKDFWAYDILLPETGKFLDWDVVLEACEFAKVKTTHEIARGTLDELLRIDPLFRSFHTPADVDGD
NVAEGFVVKQLRNEKRLHNGSRAILKVKNDKFKEKKNKAGKTPRAAVVLTEEQEKLHAAFSCYLT
ENRLRNVLSKLETVTQKQFGMISGLFIKDAKDEFERDELNETAIARDDWDVIKRSLTNIANEILRKN
WLDILDGNF.
SEQ ID NO: 15 (E15, Acidobacteriabacterium): 
MKQMYDNLKALTQKNDMFFSSIQTTPSNKPVEIFSYHIASYSDWLEPDAIECRGIMFDITDKEN
PVILSRPMEKFFNLGENPLALPEDVLPLVKTIEEKRDGSLISTYLDDGKLYTKSKGSLYSDQANDSN
RFLMRSENKDFKQALKTLAELGYTANMEYTSPKNQIVLKYEKQELIVLNIRNTETGDYLTLDDLAEL
DAENPEHTIYTHIAERFVDYEEVDGMSDEDVERIRDMKGIEGYVLVGQNQRVKLKTDWYVNLHRL
KDNINNNRRLVESVVESRTDDLRQLFEHDQGSIDKIKEFEQYVLSKIKSCLAEVRTVYERVKHLDRK
HYAINAQRYVDYTPLFSVIMRQFDDYNEENVVENIKQIALKNVDDFIPMHYK.
SEQ ID NO: 16 (E16, Vibrio phage nt-1 rnlA): 
MNELYNNLMTLAESAEGKFFFADHLSPQGEKFRVFSYHIASYSDWLLPDALEARGIMFQLDE
NDEMIRIVSRPMEKFFNLGENPFTMDLDLTTTVQLMDKADGSLISTYLTGDNFALKSKTSIYSEQAV
 
AANRYIKHVDNRDLWEFCDDCAAQNFTVNMEWCAPNNRIVLEYTEPKLIILNIRDNETGQYVSFDD
IPIGALTRIKKWLVDEYDPMTAHVDDFVETLKAKKGIEGMILRLASGQSVKIKTTWYVDLHAQKDSV
NSPKKLVTTILNKNHDDLYALFADDKPTIERIREFDDHVSKKVVESFHAVSQYYTKNRHLSRKDFAI
AGQKALKPWEFGVAMIAYQNQTVEGVVTMLINAYLKRPELLIPEKYLSEV.
SEQ ID NO: 17 (E17, Streptomyces phage Wakanda): 
MVHVSEIFSTDDLEQAIENGYVRVNPHPTAPLAILGYTEKAQFDRMWNDVTLNCRGLIIDHDG
FVVARPWKKFFNFGERPLMFDTDAPVEVTDKKDGSLGILYRDPVTGDYAISTRGSFTSDQAIHATE
LFNKKYSHIVTPFKETTFLFEIVYPKNRIVLNYGDMDDLILLGAVDNERGYYWGPRQAAGIMGWPG
PVTEVFRYRSLNECFGVHRENAEGLVIRSGSDMVKLKQADYVELHRIVTNLNERSVWSQLKDGKA
AYQICELIPDEWHEWVVDVAQALWTEYSSLENKVLGTYADLIGELPGGFTRKQFAMKATKTPYAG
YLFALLDCKSISDMLWEAVKPEVNNK.
SEQ ID NO: 18 (E18, Morganella phage vB_MmoM_MP1): 
MFKKYNSIENIDNAKLVDKVRYEGFTDPSVQWIAQEKIHGANFSFMYDGKTLKCAKRSGDIKE
TESFFGYEIILAQYKKSVLDAWDILVEHFKGVEEINIFGEFAGPGIQKEVDYEHKDFYAFDILVNGRYI
DKEIVNETCKLARIKLAPIIRYGSFDELCNMERAFDTLVPKYNEMLKKNTDAFDFIFGEQEEGKDNIS
EGYVISPVVPAHLTNGNRVIFKCKNAKFKEKGKIREVKPVKELSESDMKFVELATAYITEQRLSNVIS
HIGEIGPKDFGKVMGLMSKDVYEDMAKDGFDVLETTDIPLVKKTIQMEITTFIRKDWVNYVSR.
SEQ ID NO: 19 (E19, Flavobacterium terrae): 
MEFRKYNSIENTYQKTVLEQIIKHEFDAVEYVVQEKVHGANFAFYTNDIQVKIAKRSDFIEEND
TFYNAHKVAQKYHAKVKSLFYEVKELFPNVQFIAVFGELFGGHYNHPEVPTVQGAIKVQKGIHYSP
ENDFYAFDIKVDNSHYLNVDLANKLFEKTDFFYAKTLFKGSLADCLKYPNDFNSLIPEWLDLPTLET
NIVEGVIIRPVEALFFGNGSRVLLKNKNEKWAEKAKCDKSKKDLALVDFPIEAKPVWSKIKDYVTVN
RLYNVLSKEGEFYPKIIGKLTGWMSQDVINDFKKENPSELDTLEKESQRAINKRLNTMVIKLIKEEF
MTFEV.
SEQ ID NO: 20 (E20, Klebsiella phage vB_Kpn_F48): 
MFEKYSSLENHYNNRFIEKIRHHGYDVTEGWCAREKIHGTNFSIIIERDAVTCAKRTGPILPAE
DFFGYTVILKKYNDSIKAVQHTIKEGSSMQIFGEFAGSGIQKGVDYGEKDFYVFDILVKTDKGTHQF
VDDFMMEQMCCTFGFKVAPLMGRGSFDELAKLPNEFQINVNRYNEAAEVDLRNANTRVWPAEE
ATDNTAEGYVLKPNYPRFLANGSRVAIKCKNSKFSEKAKSDKPIKPKAVLSEIDQEVLQKFSEYVTI
QRVNNVISKIGQVGPKDFGKVMGLTVQDILVEAGREGLEVIQAEQPDIVKKEITKLVQDVLRPAWIE
LVSN.
SEQ ID NO: 21 (E21, Escherichia phage EcS1): 
MTFEKYSSLENHYNGKFIEKIRFSGNDTGVWVAREKIHGTNFSLIISRDAVTPCKRTGPILPGE
SFFAHEIIMKKYDKSIKFLQDSICGTASSYQIFGEFAGGGIQKGMDYGEKDFYVFDILIKSEHDQEGT
 
YADDFMVERMCCTFGFKVAPLIARGSFEELSQLVNDFDSVVNHYNELAVANGIEYANIAHFGFLPH
GEKNIAEGYVLKPCYPVILANGNRVAIKCKNSKFSEKAKSDKPIKPAAVLSDQDKVALSTLAEYSTW
NRVSNVLSHIGEVTAKDFGKVMGLTMQDIFVEAEREGIVLIDADQPNLVKKELQQIVMATIREKWH
EVV.
SEQ ID NO: 22 (E22, Acinetobacter phage AbTZA1): 
MSFIKYSSLENHTNDKFIQKCFEAVANGEGTVNTEFVAREKIHGTNFSVIITPDSIQAAKRSGPI
AITEKFFGYEDLMAELDDIFKEIQQDLVKNVPSFKSIQIFGEYAGGGIQKEVDYGPKSFYVFDIFMDA
PDSGFSHGWWSDVNVQAFCDYRGLKIAPLVARGTLEQLLKLPVEFDSIVPSLTWENMYSVHAQP
APKDNVAEGLVIKPNSPMFLPNGSRLAIKYKTDKFKEKGKGKAPKIPVPLSDADKELLNKLSEFSTI
ARISNVASHIGELTPKMFGKLLGMTMHDLLTEAEREGISPKSAEAPSKVKNELQKIVQVDVREFWT
SYDFPVVNVD.
SEQ ID NO: 23 (E23, Klebsiella phage KMI11): 
MFAKYSSLENHYNNKFIEKIRGAGFDMHTVEWVAREKIHGTNFSVIITPTEIVPAKRTGPILESE
SFFGHDIIMKKYKDSFVKMQNMLNAMDLVSVQIFGEFAGGGIQKGVDYGEKDFYVFDILSDSGNE
KVYWDDYVVESFATGLGLKLAPLLGRGSFAELSQYVNDFKSIVNYYNELVDTTDLEHANKHVFGP
QASSETGVAEGYVLKPVHPKFFNNGTRVAIKCKNSKFSEKAKSDKPIKAKVELTENDKRVLEIFSEY
VTWNRVSNVLSHIGTVTAKDFGRVMGLTMKDIINEAAREGHDMLFADNPSAVKKEITTLIQNTIRSK
WHEVLE.
SEQ ID NO: 24 (E24, Yasminevirus sp. GU-2018): 
MSTPPFLKYSSIESYTDVKHLEKIRQIFKSDQFTASEKVHGANFQFIVNEDASNNKISIACAKRT
SILEDSEEFYDWRNVLSKHRASLEILYQKVKTDLNKYTGDSSTKNLAGVRVYGELFGGEYPGHGK
KNLKPVQKWIYYNTDIDFIVFDVVVVINSDSPDHTSETFLSSDDVMDLICSCSSLKSDDDSVFSLKT
VPILSRGSLDEVIEFCTKNVQFNTKIPHLYGLPDIENNFAEGYVVKANVRVNVGKHRGVVKVKNPK
FDEIIGFVRHDKRDKKSTNDVSEEVKYHTEMIKPYLTQNRLDNVVSKIGPASVRPKIVGCFLNDAKI
DYIKSFDDDDSVKDFEKVWKDVQKNLTPLCNTFVYP.
SEQ ID NO: 25 (E25, Enterobacter phage vB_EclM_CIP9): 
MFEKYSSLENHYNGKFIEKIRNAGFDVTEPWVAREKIHGTNFSIIIERDAVTCAKRTGPILAGED
FFGYEIVLKKYNDSIKAVQHTIKEGSSMQVFGEFAGGGIQKGVDYGEKDFYVFDILVKTAEGTNQF
VDDYMMETICNTFGFKMAPLLGRGKFDDLIQLPNMLDVVVNDYNELAANEGIPVANKQVWKAVVA
EDNIAEGYVLKPCYPKFFPNGSRVAIKCKNSKFSEKSKSDKPIKPKVELTEQDNKVYFDFESYINVN
RINNVVSKIGKPGPKDFGKVMGLTVKDLLEEAEREGIVLTTADNPDLVKKAIIKTTQDVLRHVWQYLI
SDE.
SEQ ID NO: 26 (E26, Escherichia phage CJ20): MFKKYNSLENHYNNKFIEKIRLQGFTSGEWVAREKIHGTNFSLIIEQDNVTCAKRTGPILPAED 
FYGYEIVLKNYADSIKSVQKLLQDINYQAYQIYGEFAGAGIQKNVDYGDKDFYVFDIRVTKEDGSES
VLTDTLMEAFCIIHKFKVAPRLATGSFEDLIKLPNDLDSVVPDYNFTVDNAGLTTANTTDFDAKVEG
KVFTAEGFVLKPDVPAWLPNGERVAIKCKNSKFSEKKKSDKPIKAAVVLSQEDLDILWKFTDYVTV
NRINNVISKIGEVSPKDFGKVMGLTVQDILEEAGREGLELTQSENPVEVKKQLITNVRETLRHVWIA
LISE.
SEQ ID NO: 27 (E27, Cronobacter phage A24): 
MKFEKFQSLINESMKNVGGFLRDSSFGKRKAVVTEKIHGANFSVYFGCKDNVVGINFASRSQ
MLCPETNFMNCQRYFTEEKIQELLEHASTIMALDKCELRFIGEIYGGMDIAGIKPVQREVHYSGDIG
FRVFHAQIANNETGEVFDYNWKNVKALASDFGFEVVPIVAEGLTFEQAYALDQKVKSALSDKDQIC
EGYCIRVDADGEEGQAPLIFKKRNTDFLETKGTVKAPQNTVEQTELGMEAMARIGSFITPQRVSNV
NSHHGYSSPKDFQNLMAAVLEDVLDEYKKEYDVDLRNEPVWKEIKRSVGAQVVPLVREVLL.
SEQ ID NO: 28 (E28, Rotaria sp. Silwood1): 
MSEFEGYGKISESSSNWTLEKSDNTTFKRILWCVTEKVHGANFCFLCDNNGQRIQCGKRTSL
LSDTDEFFGYKRRLFNEIIPKIQQIYQFVQNTFPNLEKLYVFGELFGGYYPHPDVPKLPNINAIQTGI
WYCPDIEFYAFDLAISINDKQTYLDYEYAITAFQHVNLLYAEPLFIGKYEEALDFKLGFNSHIPGKLGL
PPLHEDNKAEGIVIKPMHEIMIKTNKGSPIRAIVKKKIEEFAEDKYNQAQKQVFKNDEQFSNVDLLRF
EIDALITDNRLNNALSKVGHVTRDDKEKLKELLNIYVKDIMDQLYENGNEDMWNNLSSNDRNLFTE
ELTLNAKKVILKYLKTNKC.
SEQ ID NO: 29 (E29, Acinetobacter phage Ac42): 
MPFVKYSSLENHTNSKFLEKIVEAITLGYCDNHFVAREKIHGTNFSVIITKDSIQPAKRSGPISET
ESFFGWQDLMAKYRKSFEVIQEKLIAGEYKQEIEGSPGFVPSVTVEKVQIFGEYAGGGIQKEVDYG
EKDFYAFDVMFDDKYINDVAATGVASVAGLKMAPIIAIGTLEELLKLPVEFNTTVPTYNKLWDEAGN
GFYDFVKPEAPTDNVGEGLVIKPVVPAFLGNGSRIAIKYKTDAFKEKGKGKAPKLPTPMTDKDIALA
TQFSEFITENRLSNVLSHIGEVTAKDFGKVMGLMMKDIFAEAKREEISIDVADSPSKVSKEIQHQVT
TLIRSKWVDIISN.
SEQ ID NO: 30 (E30, Helcococcus kunzii ATCC 51366): 
MKFTRYPSLTNHYAIGKTRDLIGKLDDEYYATEKNHGTNIQLTVDKNLRVGVGKRSTFITDEKP
YSDVFELSEDIIAELQGFIAENADIEQVTLYGELFGSGVQKTDYQANKDKERQVRFYDCFIHFTNGK
IIASKKDLYELVKEEFVAKIIKTGKLIDLVKEELPKESTYGGNTFEGYVYKPNVDNYVLNSNNNGIYY
PVVKHKTEDFSEVRNKVKIELGEEEIKLHNLVESYITKNRVVNVLSHGDIELIPQNIGEIIKLVQEDIKK
ELIKENPEVDKNLVYQIVRKKGSLIVPLIKKIMFEEVE.
SEQ ID NO: 31 (E31, Vibrio phage CHOED): 
MLRPFEKYNSLTLIRENHEDELQKYIDLTQGQRFVVTEKIHGANFEVATDGQTVRYASRNQVL
GEGADFYGYRFVAQFLTPKILAMFKDMKELIMEQHKACDYIEAKGDQANRPDIPRDFQEITIRGELA
 
GGQYPNAPKVKEGNGQVGKGKIWYSQQKSFFAFEICVDGVPQDFYTLGTMCGSYGIPVAPVLTF
GTFEECLEFSREHLEDITTVPNMTPLLNENGHPILNEDSSYCALHPIHNNTREGHVISSVNPVYLPN
GKRMVFKHKGVKFMENKGEKKPKAKVEVILTPEQQVVFDTILPMITVARMEAVMSKEGTWEPKDF
TKLCGKVLEDATDEAHGDAPNAFFAAWAHISTKERKVVMKQLGREAAERLRTAYFS.
SEQ ID NO: 32 (E32, Cronobacter phage S13): 
MFKKYNSLTNHTDKKFLNKILFAGLTDPKVLWVAREKIHGANFSFIVTSDSVSVGKRSGVIPEG
ENFYSHADIESRYRADVISVRRRLIDLGIATETSEIQIYGELAGVTSTGKLIQHGVDYGPSDFYVFDIT
VDGVYQDDTTCVLALTRTNITMAPFLKMGTFDELISMRNDFDSVVQRWKNDFNAIVWDKGEHLQY
TETNTAEGFVMKPTEVHYVGMERVAIKHKNEKFSEKKNKVKNFKAPVPMTEKDQEVLDELLTFNT
ENRVRNVLSKTGTPDTKQFGMIMGLTVKDILEESKNILDTADAPATVKKTLIAEVSNLIRPHWVNICN
GEF.
SEQ ID NO: 33 (E33, Thecamonas trahens ATCC 50062): 
MEGMVEAVAAAFVPYPKIPTAFSGSADDARRVRTIEWVATEKIHGANLTLMCDGQVVVPAKR
RGVLADDEDFFGLRSSGLLASLARPAIELAQALGAGSGTSIAVSGELFGADVQPGIDYGPQLQFAV
FDVALLAADGDRRFLAHDEMTAVATAAGFTVVHEVARGSYTKLLDTPIGFDSHWPALLGLEPLPPA
GANTAEGIVLRPATTDDRFLVKIKDPSFADVEASGRAHKSMMVAADGSSSAAADAQNIFYELQLYL
TPARAQSARDW.
SEQ ID NO: 34 (E34, Acinetobacter phage vB_AbaM_phiAbaA1): 
MMKFIKYPSIKRLTERAIHELDKLPFDLPVFVVTEKLHGANMGIYCDGETIRIAKREGFQDECET
KNFFDSARVAEKYSESILGLVRSWNLVKKSHGLEGYAYYIFLGELFGGGIHKGTPYSMEQDFALFN
IFTVIDDTEANREYVKKLCAAYFEDEVNGKLVLMPQSRERIVNIAKYINCKVLDILFKGTLEECAQFA
NDFESHFVCPNRVRESGVEKHGDDAVKNLCITEGVIIEPFYYTNYKGNNFTYKSKNAKFEEKYATG
YKPKVSLVQKAIDNLTEDQAEMLYTLNEYLVMPRYQSVVSKMGEVTIKDFSKVLKAFVEDVLKDAK
EDFAEDGKEFVKPTKDMMMLFTNEVKSYIRPILLGE.
SEQ ID NO: 35 (E35, Klebsiella phage Mineola): 
MFAKYSSLENHYNGKFIEKIRGAGFDMHTVEWVAREKIHGTNFSVIITPTEIVPAKRTGPILESE
SFFGHDIIMKKYKDSFVKMQNMLNAMDLVSVQIFGEFAGGGIQKGVDYGEKDFYVFDILSDSGNE
KVYWDDYVVESFATGLGLKLAPLLGRGSFAELSQYVNDFKSIVNYYNELVDTTDLEHANKHVFGP
QASSETGVAEGYVLKPVHPKFFNNGTRVAIKCKNSKFSEKAKSDKPIKAKVELTENDKRVLEIFSEY
VTWNRVSNVLSHIGTVTAKDFGRVMGLTMKDIINEAAREGHDMLFADNPSAVKKEITTLIQNTIRSK
WHEVLE.
SEQ ID NO: 36 (E36, Enterococcus phage 9181): 
MPFFKYPSLTNEYAVAKSRSILSKMNTIAYSTEKIHGSNVQYVFDDRDDVVNFGAFSRNGKKV
GGKDDNGEHIVDTQFKLVMDIAFQMDLPNIASSIFTEFNHVDSINIYGEYFGSGVQRTQYFINERNE
 
RGFQVFDIFLHYKGDPQEVYRSMGLLELIDFVGRDKMVPIEDIVFLSNLIEKDIVERSHYGGEREGN
VYKEIHGSWIDPVTKYCGIKHKRPEFLEVSKAPKQPKAKLSDEFLEVVERLSTYVTENRVRNIHSH
GDIKFITKNIGAIMLEVKKDILKEFEDNEESIPNVDLSKAIRACDRDIALAVRKVIAEVGDMED.
SEQ ID NO: 37 (E37, Aeromonas phage Ahp1_CNU-2021): 
MIFKSYPEMENHSKSRIINAVIEQGHGAADVVWCAREKIHGTNFSLWYNGVDDVRAARRTDF
LEFADKFYDFTNIERRYQLCVQALYLRLGLTTEVMAVYGEYAGIMSSGKWIQTGIEYGPQDFYAFD
VMVDGVPIDDDVMESACLDCGMKTAPLLKVGTFKEVMGVPNDLQSVVMTKNETGTFEIVKGTDNI
AEGYVAKPRVGRRFWNGARVAIKCKNEKWSETAKGKGVKIEVKDISDKDASILAMLGDYITENRLK
NVLSKIGVPETKEFGKVMGMLSQDIIKDYESDTETLVKDADEPSRVCRMMNTAVGNFIRPLWVDIC
DGAW.
SEQ ID NO: 38 (E38, Megavirus chiliensis): 
MIIFWKDTYIYYDIIKNLKKNNIECTDYIIYIITTMNMEINQFFQKYFSIENLNARSFKSLQKNGLLS
NNIEWIALEKIHGANFSFITNGEKLETAKRTGIISSEEYFFNYQIVVERYSQDVFNIFNKIKKNIDNLMS
IQIYGELFGGAYPDMYVKDIKPIQKGVYYNPDIDFLIFDIKINYKTESGIPENNYSEFLSHDEVINYISK
TNIRIVPEISRAIFTDIIKMDPVFHTKVPAMYGLPQINNNLAEGYVFKMNARHLCNIFRPIIKSKNDDIF
GEVHKSSIKYDIKDLLLKNSEIGSSYEEIKCYLTKNRFDGIIGKIGPDNKPAKIIGCFIADALREYEITLD
ADKKIEYNKNKRNISKNITIYISNNPEIQSWFK.
SEQ ID NO: 39 (E39, Acanthamoeba polyphaga moumouvirus): 
MELAKNFQKYFSIENPKKIIKSLEKNDYTNDKYEWIVLEKIHGANFSFITNGETIYAAKRTAIIQP
GEYFYDYESIVEKYKEDVFKVYNEIFYKTKNIMSIQIFGELFGGLYPNYDKKINPIQTGIYYNPKIDF
MVFDIKINYLTDNFNVQNDKSEFLSHDEVLFYLSTTKFKHVPIIGRGQFNDIIKMEPVFLLKYLEYMN
YLMLIIIMLKGIYLN.
SEQ ID NO: 40 (E40, Salmonella phage S16):

MFKKYSSLENHYNNKFISKIRFEGKDGGLWVAREKIHGTNFSIIVSKDSVSACKRSGPILPSES
FYGHEIILKNYDESIKTIQRCMNANELGSVSSYQIFGEFAGQGIQKEVDYGEKDFYVFDILVNTQNG
NVLYMDDMMMTSFCNEFGFKMAPFIGCGSFDELIQLPNNFTSVIKAYNEAAKDDLKEVNLCVFDPL
VTDDNVAEGYVLKPVYPDFFNNGTRIAIKSKNSRFTEKKKSDKPIKPKAVLTSNDSTVLANLCEYST
WNRVSNVISHIGEVKAKDFGKVMGLTIQDIFIEAKREGVDIVHADNPDLVKRELQTVVSNTIREKWL
EIVS.

Identity in the present application refers to "identity" between amino acid sequences or nucleic acid sequences, that is, the total of ratios of the amino acid residues or nucleotides of the same type in the amino acid sequences or nucleic acid sequences. The identity of the amino acid sequences or nucleic acid sequences may be determined with comparison programs such as a Basic Local Alignment Search Tool (BLAST), FASTA, etc.

Active sites, active pockets, active mechanisms, protein structures, etc. of proteins having greater than 70%, 75%, 80%, 85%, 90%, 95%, 99% (e.g., greater than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even greater than 99.9%) identity and having the same functions are probably the same as the protein provided the sequence in a).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

For rules of substitution, replacement, etc., if amino acids are similar in nature, replacements have similar effects. For example, in the homologous protein, conservative amino acid replacements may occur. "Conservative amino acid replacement" includes, but is not limited to:
Hydrophobic amino acids (Ala, Cys, Gly, Pro, Met, Val, Ile, and Leu) are substituted by other hydrophobic amino acids.

Hydrophobic amino acids with bulky side chains (Phe, Tyr, and Trp) are substituted by other hydrophobic amino acids with bulky side chains.

Amino acids with positively charged side chains (Arg, His, and Lys) are substituted by other amino acids with positively charged side chains.

Amino acids with polarized and uncharged side chains (Ser, Thr, Asn, and Gln) are substituted by other amino acids with polarized and uncharged side chains.

Person skilled in the art may also make conservative substitutions of the amino acids according to amino acid substitution rules known to person skilled in the art, such as a "blosum62 scoring matrix" in the prior art.

In a preferred embodiment, the sense strand substrate includes 2 or more substrates, and the antisense strand substrate includes 2 or more substrates. Preferably, the sense strand substrate has a length of 2-19 nt, more preferably 3-17 nt, and further preferably 6-15 nt. Preferably, the antisense strand substrate has a length of 2-21 nt, more preferably 3-19 nt, and further preferably 6-15 nt.

In a preferred embodiment, both the sense strand substrate and the antisense strand substrate include 2 substrates, where the sense strand substrate includes a first sense strand substrate and a second sense strand substrate, and the antisense strand substrate includes a first antisense strand substrate and a second antisense strand substrate. The method includes: the first sense strand substrate, the second sense strand substrate, the first antisense strand substrate, and the second antisense strand substrate are mixed, the RNA ligase is utilized to catalyze the ligation between the first sense strand substrate and the second sense strand substrate to form the sense strand, and to catalyze the ligation between the first antisense strand substrate and the second antisense strand substrate to form the antisense strand, and the sense strand and the antisense strand are complementary paired with each other to form the siRNA. Preferably, the sense strand substrate and the antisense strand substrate are annealed, and then mixed with the RNA ligase, so as to obtain the siRNA.

In the above method, the sense strand substrate and the antisense strand substrate may be mixed and annealed first. A double-stranded RNA structure can be formed between the sense strand substrate and the antisense strand substrate through complementary base pairing, and nicks among different substrates exist in the double-stranded RNA structure. Then, the annealed reaction system is mixed with the RNA ligase. The phosphate group and the hydroxyl group on both sides of the nick are ligated by the RNA ligase to form the phosphodiester bond using, thereby repairing the nick, and thereby obtaining the target product siRNA with the complete double-stranded structure.

In a preferred embodiment, the 3' end of the first sense strand substrate is ligated to the 5' end of the second sense strand substrate under the catalysis of the RNA ligase, forming the sense strand; and the 3' end of the first antisense strand substrate is ligated to the 5' end of the second antisense strand substrate under the catalysis of the RNA ligase, forming the antisense strand. Preferably, in the above method, the 5' end of the first sense strand substrate is a 5' protecting group, and the 3' end of the first sense strand substrate is a hydroxyl group; and the 5' end of the second sense strand substrate is a phosphate group, and the 3' end of the second sense strand substrate is an L96 group (N-acetylgalactosamine group derivative). The 5' end of the first antisense strand substrate is a hydroxyl group, and the 3' end of the first antisense strand substrate is a hydroxyl group; and the 5' end of the second antisense strand substrate is a phosphate group, and the 3' end of the second antisense strand substrate is a hydroxyl group. The structure of the above L96 group is shown in Fig. 2.

In a preferred embodiment, the first sense strand substrate is a nucleic acid as set forth in SEQ ID NO: 41, and the second sense strand substrate is a nucleic acid as set forth in SEQ ID NO: 42; or the first sense strand substrate is a nucleic acid as set forth in SEQ ID NO: 53, and the second sense strand substrate is a nucleic acid as set forth in SEQ ID NO: 54.

In a preferred embodiment, the first antisense strand substrate is a nucleic acid as set forth in SEQ ID NO: 44, and the second antisense strand substrate is a nucleic acid as set forth in SEQ ID NO: 43; or the first antisense strand substrate is a nucleic acid as set forth in SEQ ID NO: 56, and the second antisense strand substrate is a nucleic acid as set forth in SEQ ID NO: 55.

The siRNA can be prepared with the above method and the substrates shown in SEQ ID NOs: 41-44 or 53-56. However, it is to be noted that, the selection of substrates is not limited to those shown in SEQ ID NOs: 41-44 or 53-56. Any substrates capable of forming the sense strand and the antisense strand can be applied to the above method. The above method is suitable for the preparation of the siRNA but is not limited to different ligation positions of the substrates. The above preparation has a good ligation effect on the ligation of the sense strand sequence and antisense strand sequence of the siRNA. The number of the sense strand substrates or antisense strand substrates includes, but is not limited to, 2, 3, 4, or even more.

In a preferred embodiment, both the sense strand substrate and the antisense strand substrate include 3 substrates, the sense strand substrate includes a first sense strand substrate, a second sense strand substrate, and a third sense strand substrate; and the antisense strand substrate includes a first antisense strand substrate, a second antisense strand substrate, and a third antisense strand substrate. Preferably, the first sense strand substrate is a nucleic acid as set forth in SEQ ID NO: 47; the second sense strand substrate is a nucleic acid as set forth in SEQ ID NO: 48; and the third sense strand substrate is a nucleic acid as set forth in SEQ ID NO: 49. Preferably, the first antisense strand substrate is a nucleic acid as set forth in SEQ ID NO: 52; the second antisense strand substrate is a nucleic acid as set forth in SEQ ID NO: 51; and the third antisense strand substrate is a nucleic acid as set forth in SEQ ID NO: 50.

In a preferred embodiment, the concentrations of the sense strand substrate and the antisense strand substrate are both 1 µM to 1M. Preferably, the concentration of the RNA ligase is 0.01 mg/mL to 10 mg/mL. Preferably, in the reaction system formed by mixing the sense strand substrate, the antisense strand substrate and the RNA ligase, ATP, Tris-HCl, MgCl₂, and DTT are further included. Preferably, the reaction temperature of the method is 0-40 °C, more preferably 15-30 °C. Preferably, the reaction time of the method is 0.1-48 h, more preferably 12-24 h.

The beneficial effects of the present application are further described in detail below with reference to specific embodiments.

### Embodiment 1 Purification and preparation of RNA ligase

A gene that could express an RNA ligase was constructed into a pET28a vector, and transformed into an *E*. *coli* BL21(DE3) expression strain. The expression strain was inoculated in a 5mL LB culture medium (containing 50 µg/mL) based on a 0.1% inoculation amount, and cultured for 16 h at 37 °C. A culture solution obtained was transferred into a 500mL LB culture medium based on a 1% inoculation amount, culture was performed at 37 °C until OD₆₀₀ = 0.6, 0.1 mM IPTG was added, and induction was performed for 16 h at 20 °C. An induced culture solution obtained was centrifuged to obtain bacterial sludge. The bacterial sludge was resuspended with a lysis buffer (50 mM Tris-HCl pH8.0, 0-500 mM NaCl, 10% glycerol) to cause a final concentration of the bacteria to be 10%; and ultrasonication was performed, and a crude enzyme solution was obtained through centrifugation. 10 mM of imidazole was added to the crude enzyme solution; an enzyme solution obtained through filtration using a 0.22 µm filter membrane was purified with a nickel column; desalination and solution exchange was performed, and then secondary purification was performed with a strong anion column; and the obtained enzyme solution was subjected to desalination and solution exchange and then stored in 30% glycerol.

### Embodiment 2

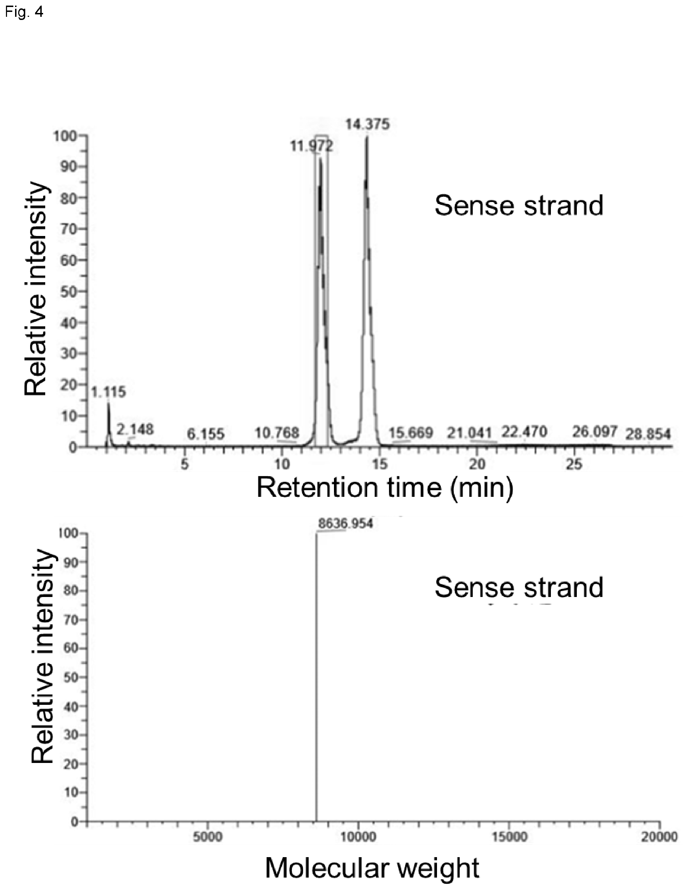

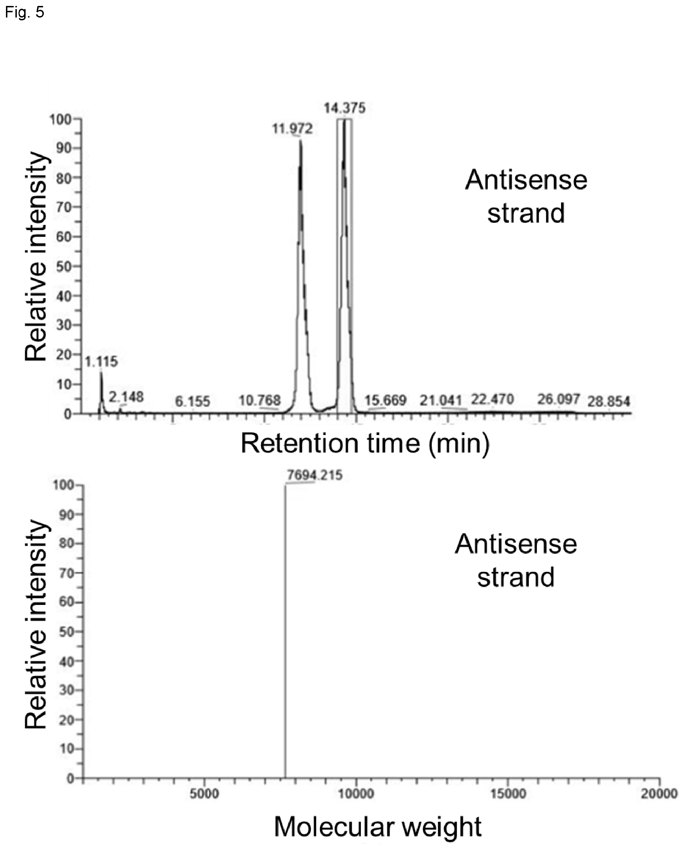

Substrates 1-4 were added to a clean reagent bottle to prepare a reaction system, causing a final volume to be 100 µL; and sequences of the substrates 1-4 were shown in Table 1. The concentrations of RNA fragments 1-4 (i.e., the substrates 1-4) were all 50 µM, the concentration of ATP was 1 mM, the concentration of Tris-HCl (pH 7.5@25 °C) was 50 mM, the concentration of MgCl₂ was 10 mM, the concentration of DTT was 1 mM. A commercial ligase T4 RNA2 (E4), a T7 DNA ligase (E5) or other ligases (E1-E3, E6-E16) were added respectively to cause the concentration of the enzyme to be 0.2 mg/mL. The reaction system underwent a reaction for 16 h at 16 °C. The obtained reaction system was held for 5 min at 80 °C to deactivate the ligase, a sediment was removed through centrifugation at 12000 rpm, supernatant was detected through SDS-PAGE, and SDS-PAGE results were shown in Fig. 3. Results showed that a portion of the ligases could catalyze the synthesis of the final product. The theoretical molecular weight of the sense strand was 8636.95±1, and the theoretical molecular weight of the antisense strand was 7694.20±1. Through mass spectrometry identification, results showed that the molecular weight conformed to that of the final product. Liquid chromatography and mass spectrometry detection results were shown in Fig. 4 and Fig. 5. Fig. 4 was a liquid chromatogram diagram of the siRNA and a diagram of mass spectrometry results for the siRNA sense strand. Fig. 5 was a liquid chromatogram diagram of the siRNA and a diagram of mass spectrometry results for the siRNA antisense strand.

In Fig. 3, Lane 1 was a reaction system catalyzed by E1; Lane 2 was a nucleic acid molecular weight standard; Lane 3 was a reaction system catalyzed by E2; Lane 4 was a reaction system catalyzed by E3; Lane 5 was a reaction system catalyzed by E4; Lane 6 was a reaction system catalyzed by E5; Lane 7 was a reaction system catalyzed by E6; Lane 8 was a reaction system catalyzed by E7; Lane 9 was a reaction system catalyzed by E8; Lane 10 was a negative control reaction system without adding an enzyme; Lane 11 was a reaction system catalyzed by E9; Lane 12 was a reaction system catalyzed by E10; Lane 13 was a reaction system catalyzed by E11; Lane 14 was a reaction system catalyzed by E12; Lane 15 was a reaction system catalyzed by E13; Lane 16 was a reaction system catalyzed by E14; Lane 17 was a reaction system catalyzed by E15; and Lane 18 was a reaction system catalyzed by E16.

**Table 1**

| Substrate | Sequence (5'-3') | 5' end | 3' end |
|---|---|---|---|
| Substrate 1 (SEQ ID NO: 41) | Cms-Ums-Am-Gm-Am-Cm-Cf-Um-Gf-Um-dT-Um-Um-G m | Hydroxyl group | Hydroxyl group |
| Substrate 2 (SEQ ID NO: 42) | Cm-Um-Um-Um-Um-Gm-Um | Phosphate group | L96 |
| Substrate 3 (SEQ ID NO: 43) | Af-Gm-Gf-Um-Cf-Um-Am-Gms-Ams-Am | Phosphate group | Hydroxyl group |
| Substrate 4 (SEQ ID NO: 44) | Ams-Cfs-Am-Af-Af-Af-Gm-Cf-Am-Af-Am-Af-Cm | Hydroxyl group | Hydroxyl group |

The m after A, C, G, or U represented 2' methoxy modification for the ribonucleotide, the f represented 2' fluoro modification for the ribonucleotide, the s represented the phosphorothioate bond, the d before A, C, G, or T represented that the nucleotide was deoxyribonucleotide, and L96 was the N-acetylgalactosamine group derivative shown in Fig. 2.

The ribonucleotides at positions 1, 2, 3, 4, 5, 6, 8, 10, 12, 13, and 14 of the substrate 1 had 2' methoxy modifications; the ribonucleotides at positions 7 and 9 had 2' fluoro modifications; the ribonucleotides at positions 1 and 2 were ligated by a phosphorothioate bond, the ribonucleotides at positions 2 and 3 were ligated by a phosphorothioate bond; and the position 11 of sense strand was deoxyribonucleotide, which was a thymine (T).

The ribonucleotides at positions 1-7 of the substrate 2 all had 2' methoxy modifications.

The ribonucleotides at positions 2, 4, 6, 7, 8, 9, and 10 of the substrate 3 had 2' methoxy modifications; the ribonucleotides at positions 1, 3 and 5 had 2' fluoro modifications; the ribonucleotides at positions 8 and 9 were ligated by a phosphorothioate bond, and the ribonucleotides at positions 9 and 10 were ligated by a phosphorothioate bond.

The ribonucleotides at positions 1, 3, 7, 9, 11, and 13 of the substrate 4 had 2' methoxy modifications; the ribonucleotides at positions 2, 4, 5, 6, 8, 10, and 12 had 2' fluoro modifications; and the ribonucleotides at positions 1 and 2 were ligated by a phosphorothioate bond, and the ribonucleotides at positions 2 and 3 were ligated by a phosphorothioate bond.

The sense strand (SS) of the siRNA obtained through preparation was 5'-CmsUmsAmGmAmCmCfUmGfUmdTUmUmGmCmUmUmUmUmGmUm-3' (SEQ ID NO: 45).

The antisense strand (AS) was 5'-AmsCfsAmAfAfAfGmCfAmAfAmAfCmAfGmGfUmCfUmAmGmsAmsAm -3' (SEQ ID NO: 46).

The 5' end of the above sense strand was a hydroxyl group, and the 3' end of the sense strand was an L96 group, the ribonucleotides at positions 1, 2, 3, 4, 5, 6, 8, 10, 12, 13, 14, 15, 16, 17, 18, 19, 20, and 21 of the sense strand had 2' methoxy modifications; the ribonucleotides at positions 7 and 9 had 2' fluoro modifications; the ribonucleotides at positions 1 and 2 were ligated by a phosphorothioate bond, the ribonucleotides at positions 2 and 3 were ligated by a phosphorothioate bond; and the position 11 of sense strand was deoxyribonucleotide, which was a thymine (T).

The 5' end of the above antisense strand was a hydroxyl group, and the 3' end of the antisense strand was a hydroxyl group, the ribonucleotides at positions 1, 3, 7, 9, 11, 13, 15, 17, 19, 20, 21, 22 and 23 of the antisense strand had 2' methoxy modifications; the ribonucleotides at positions 2, 4, 5, 6, 8, 10, 12, 14, 16 and 18 had 2' fluoro modifications, the ribonucleotides at positions 1 and 2 were ligated by a phosphorothioate bond, the ribonucleotides at positions 2 and 3 were ligated by a phosphorothioate bond, the ribonucleotides at positions 21 and 22 were ligated by a phosphorothioate bond and the ribonucleotides at positions 22 and 23 were ligated by a phosphorothioate bond.

### Embodiment 3

Substrates 1-4 were added to a clean reagent bottle to prepare a reaction system, causing a final volume to be 100 µL. The concentrations of RNA fragments 1-4 were all 200 µM, the concentration of ATP was 1 mM, the concentration of Tris-HCl (pH 7.5@25 °C) was 50 mM, the concentration of MgCl₂ was 10 mM, the concentration of DTT was 1 mM. One or more of a commercial ligase T4 RNA2, a T7 DNA ligase, and the above RNA ligase were added to cause the concentration of the enzyme to be 0.2 mg/mL. The reaction system underwent a reaction for 16 h at 16 °C. The obtained reaction system was held for 5 min at 80 °C to deactivate the ligase, a sediment was removed through centrifugation at 12000 rpm. For ease of quantitation, a UPLC method was developed to accurately detect the reaction, such that supernatant was detected through UPLC. Results were shown in Table 2 and showed that a portion of the ligases could catalyze the synthesis of the final product.

**Table 2**

| Enzyme | Activity | |
|---|---|---|
| | Sense strand product | Antisense strand product |
| E1 | - | × |
| E2 | - | × |
| E3 | - | × |
| E4 | + | + |
| E5 | ++ | ++ |
| E6 | +++ | +++ |
| E7 | +++ | +++ |
| E8 | +++ | +++ |
| E9 | +++ | +++ |
| E10 | +++ | +++ |
| E11 | × | × |
| E12 | × | × |
| E13 | +++ | +++ |
| E14 | +++ | +++ |
| E15 | + | - |
| E16 | ++ | ++ |
| E17 | +++ | +++ |
| E18 | +++ | +++ |
| E19 | +++ | +++ |
| E20 | +++ | +++ |
| E21 | +++ | +++ |
| E22 | +++ | +++ |
| E23 | +++ | +++ |
| E24 | +++ | +++ |
| E25 | +++ | +++ |
| E26 | +++ | +++ |
| E27 | +++ | +++ |
| E28 | +++ | +++ |
| E29 | +++ | +++ |
| E30 | +++ | +++ |
| E31 | +++ | +++ |
| E32 | +++ | +++ |
| E33 | +++ | +++ |
| E34 | +++ | +++ |
| E35 | +++ | +++ |
| E36 | +++ | +++ |
| E37 | +++ | +++ |
| E38 | +++ | +++ |
| E39 | +++ | +++ |
| E40 | +++ | +++ |

In the indication of activity, "-" indicated that the purity of the product system was 0.1-5% (excluding an endpoint value of 5%); "+" indicated that the purity of the product system was 5%-20% (excluding an endpoint value of 20%); "++" indicated that the purity of the product system was 20%-30% (excluding an endpoint value of 30%); "+++" indicated that the purity of the product system was 30%-50%; and "×" indicated that no product was produced. In the present application, "the purity of the product system" indicated that in the reaction system, a ratio of a target product to a total component (target product + remaining substrate) in the reaction system might be calculated by the area under the curve of different components in liquid chromatography results.

### Embodiment 4

Substrates 1-4 were added to a clean reagent bottle to prepare a reaction system, causing a final volume to be 100 µL. The concentrations of RNA fragments 1-4 were the same, which were 50µM-1000µM; the concentration of ATP was 1 mM, the concentration of Tris-HCl (pH 7.5@25 °C) was 50 mM, the concentration of MgCl₂ was 10 mM, the concentration of DTT was 1 mM; and a ligase was added to cause the concentration of the enzyme to be 0.2 mg/mL. The reaction system underwent a reaction for 16 h at 16 °C. The obtained reaction system was held for 5 min at 80 °C to deactivate the ligase, a sediment was removed through centrifugation at 12000 rpm, and supernatant was detected through UPLC. Results were shown in Table 3, and showed that the optimal ligase could catalyze the concentration of the substrate to reach 1 mM, which significantly outperformed that of the commercial enzyme T4 RNA ligase 2.

**Table 3**

| Enzyme | Substrate concentration | Activity | |
|---|---|---|---|
| | | Sense strand product | Antisense strand product |
| E29 | 50µM | +++ | +++ |
| | 200µM | +++ | +++ |
| | 500µM | +++ | +++ |
| | 1000µM | +++ | +++ |
| E18 | 50µM | +++ | +++ |
| | 200µM | +++ | +++ |
| | 500µM | +++ | +++ |
| | 1000µM | +++ | +++ |
| E10 | 50µM | +++ | +++ |
| | 200µM | +++ | +++ |
| | 500µM | +++ | +++ |
| | 1000µM | ++ | ++ |
| E7 | 50µM | +++ | +++ |
| | 200µM | +++ | +++ |
| | 500µM | +++ | +++ |
| | 1000µM | ++ | ++ |
| E14 | 50µM | +++ | +++ |
| | 200µM | +++ | +++ |
| | 500µM | +++ | +++ |
| | 1000µM | ++ | ++ |
| E9 | 50µM | +++ | +++ |
| | 200µM | +++ | +++ |
| | 500µM | ++ | ++ |
| | 1000µM | - | - |
| E6 | 50µM | +++ | +++ |
| | 200µM | +++ | +++ |
| | 500µM | ++ | ++ |
| | 1000µM | + | + |
| E4 | 50µM | +++ | +++ |
| | 200µM | + | + |
| | 500µM | - | - |
| | 1000µM | - | × |
| Negative control | 50µM | × | × |

In the indication of activity, "-" indicated that the purity of the product system was 0.1-5%; "+" indicated that the purity of the product system was 5%-20% (excluding an endpoint value of 5%); "++" indicated that the purity of the product system was 20%-30% (excluding an endpoint value of 20%); "+++" indicated that the purity of the product system was 30%-50% (excluding an endpoint value of 30%); and "x" indicated that no product was produced.

### Embodiment 5

Substrates 1-4 were added to a clean reagent bottle to prepare a reaction system, causing a final volume to be 100 µL. The concentrations of RNA fragments 1-4 all were all 800 µM; the concentration of ATP was 2 mM, the concentration of Tris-HCl (pH 7.5@25 °C) was 50 mM, the concentration of MgCl₂ was 10 mM, the concentration of DTT was 1 mM; and a ligase was added to cause the concentration of the enzyme to be 0.2 mg/mL. The reaction system underwent a reaction for 16 h at 16 °C. The obtained reaction system was partially taken and held for 5 min at 80 °C to deactivate the ligase, a sediment was removed through centrifugation at 12000 rpm, and supernatant was detected through UPLC. Results showed that complete catalysis had realized. Thermal inactivation was performed on the reaction system, the ligase was removed by performing centrifugation for 30 min at 12000 rpm, the reaction system was subjected to ion column purification to obtain a purified product, and the product underwent freeze-drying after desalination, so as to obtain the final product. The determination of the final product includes water content, endotoxin residue, enzyme residue, solvent residue, metal residue, and host cell protein residue. 1.4 g of the product was obtained through weighing, the yield was 80%, the moisture content was 9%, the denaturation method purity of the product was 96%, the non-denaturation method purity was 96%, the host protein residues were 0.2 ppm, the enzyme residues were < 6 ppm, and the metal residues included: Ni: 7 ppm; Cr: 3 ppm; Fe: 43 ppm; Ti: 4 ppm; Mn: 1 ppm; Na: 31169 ppm; As: ND; Cd: ND; Hg: ND; Pb: ND. All tests met the quality standards for injectable drugs. The endotoxin residues were 9 EU/mg. Since the operation was conducted in a non-clean laboratory environment, there was a risk of environmental endotoxins contaminating the product. Therefore, the product underwent endotoxin removal in a clean environment, resulting in a final endotoxin residue of 0.2 EU/mg, which met the quality standards for siRNA drugs. It indicated that the enzymatic synthesis route for the siRNA drug was feasible, and had extremely high product yields and an exceptionally simple process.

### Embodiment 6

Substrates 5-10 were added to a clean reagent bottle; sequences of the substrates 5-10 were shown in Table 4; a reaction system was prepared, causing a final volume to be 200 µL. The concentrations of the substrates were all 500 µM; the concentration of ATP was 1 mM, the concentration of Tris-HCl (pH 7.5@25 °C) was 50 mM, the concentration of MgCl₂ was 10 mM, the concentration of DTT was 1 mM; and a ligase was added to cause the concentration of the enzyme to be 0.2 mg/mL. The reaction system underwent a reaction for 16 h at 16 °C. The obtained reaction system was held for 5 min at 80 °C to deactivate the ligase, a sediment was removed through centrifugation at 12000 rpm, and supernatant was detected through UPLC. Results were shown in Table 5. Results showed that the generation of products could be detected.

**Table 4**

| Substrate | Sequence (5'-3') | 5' end | 3' end |
|---|---|---|---|
| Substrate 5 (SEQ ID NO: 47) | Cms-Ums-Am-Gm-Am-Cm-Cf-U m | Hydroxyl group | Hydroxyl group |
| Substrate 6 (SEQ ID NO: 48) | Gf-Um-dT-Um-Um-Gm-Cm-Um | Phosphate group | Hydroxyl group |
| Substrate 7 (SEQ ID NO: 49) | Um-Um-Um-Gm-Um | Phosphate group | L96 |
| Substrate 8 (SEQ ID NO: 50) | Um-Cf-Um-Am-Gms-Ams-Am | Phosphate group | Hydroxyl group |
| Substrate 9 (SEQ ID NO: 51) | Am-Af-Am-Af-Cm-Af-Gm-Gf | Phosphate group | Hydroxyl group |
| Substrate 10 (SEQ ID NO: 52) | Ams-Cfs-Am-Af-Af-Af-Gm-Cf | Hydroxyl group | Hydroxyl group |

The m after A, C, G, or U represented 2' methoxy modification for the ribonucleotide, the f represented 2' fluoro modification for the ribonucleotide, the s represented the phosphorothioate bond, the d before A, C, G, or T represented that the nucleotide was deoxyribonucleotide, and L96 represented the N-acetylgalactosamine group derivative.

The ribonucleotides at positions 1, 2, 3, 4, 5, 6, and 8 of the substrate 5 had 2' methoxy modifications; the ribonucleotide at position 7 had 2' fluoro modifications; and the ribonucleotides at positions 1 and 2 were ligated by a phosphorothioate bond, and the ribonucleotides at positions 2 and 3 were ligated by a phosphorothioate bond.

The ribonucleotides at positions 2, 4, 5, 6, 7, and 8 of the substrate 6 had 2' methoxy modifications; the ribonucleotide at position 1 had 2' fluoro modification; and the ribonucleotide at position 3 was the deoxyribonucleotide, which was the thymine (T).

The ribonucleotides at positions 1, 2, 3, 4, and 5 of the substrate 7 had 2' methoxy modifications.

The ribonucleotides at positions 1, 3, 4, 5, 6, and 7 of the substrate 8 had 2' methoxy modifications; the ribonucleotide at position 2 had 2' fluoro modification; the ribonucleotides at positions 5 and 6 were ligated by a phosphorothioate bond, and the ribonucleotides at positions 6 and 7 were ligated by a phosphorothioate bond.

The ribonucleotides at positions 1, 3, 5, and 7 of the substrate 9 had 2' methoxy modifications, and the ribonucleotides at positions 2, 4, 6 and 8 had 2' fluoro modifications.

The ribonucleotides at positions 1, 3, and 7 of the substrate 10 had 2' methoxy modifications; the ribonucleotides at positions 2, 4, 5, 6, and 8 had 2' fluoro modifications; and the ribonucleotides at positions 1 and 2 were ligated by a phosphorothioate bond, and the ribonucleotides at positions 2 and 3 were ligated by a phosphorothioate bond.

**Table 5**

| Enzyme | Activity | |
|---|---|---|
| | Sense strand product | Antisense strand product |
| E1 | × | × |
| E2 | × | × |
| E3 | × | × |
| E4 | + | + |
| E5 | + | + |
| E6 | +++ | +++ |
| E7 | +++ | +++ |
| E8 | +++ | +++ |
| E9 | +++ | +++ |
| E10 | +++ | +++ |
| E11 | × | × |
| E12 | × | × |
| E13 | +++ | +++ |
| E14 | +++ | +++ |
| E15 | × | - |
| E16 | ++ | ++ |
| E17 | +++ | +++ |
| E18 | +++ | +++ |
| E19 | +++ | +++ |
| E20 | +++ | +++ |
| E21 | +++ | +++ |
| E22 | +++ | +++ |
| E23 | +++ | +++ |
| E24 | +++ | +++ |
| E25 | +++ | +++ |
| E26 | +++ | +++ |
| E27 | +++ | +++ |
| E28 | +++ | +++ |
| E29 | +++ | +++ |
| E30 | +++ | +++ |
| E31 | +++ | ++ |
| E32 | ++ | +++ |
| E33 | +++ | +++ |
| E34 | +++ | +++ |
| E35 | +++ | +++ |
| E36 | ++ | ++ |
| E37 | +++ | +++ |
| E38 | +++ | +++ |
| E39 | + | ++ |
| E40 | +++ | ++ |

In the indication of activity, "-" indicated that the purity of the product system was 0.1-5%; "+" indicated that the purity of the product system was 5%-20% (excluding an endpoint value of 5%); "++" indicated that the purity of the product system was 20%-30% (excluding an endpoint value of 20%); "+++" indicated that the purity of the product system was 30%-50% (excluding an endpoint value of 30%); and "x" indicated that no product was produced.

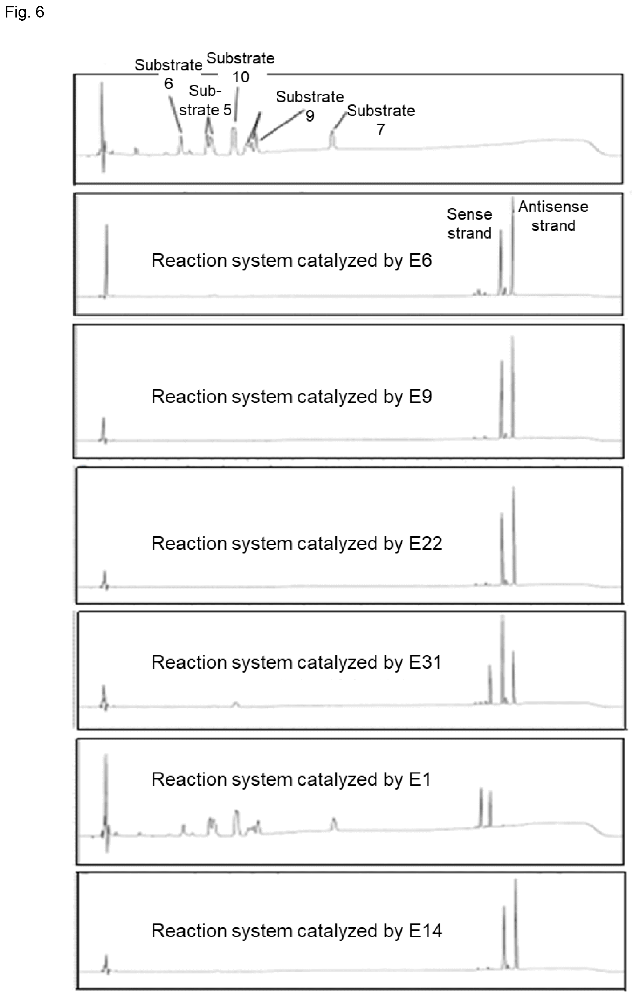

Fig. 6 showed a UPLC detection diagram of an enzyme-free system and a reaction system catalyzed by partial enzymes.

### Embodiment 7

Substrates 11-14 were added to a clean reagent bottle; sequences of the substrates 11-14 were shown in Table 6; a reaction system was prepared, causing a final volume to be 200 µL. The concentrations of the substrates were all 500 µM; the concentration of ATP was 1 mM, the concentration of Tris-HCl (pH 7.5@25 °C) was 50 mM, the concentration of MgCl₂ was 10 mM, the concentration of DTT was 1 mM; and a ligase was added to cause the concentration of the enzyme to be 0.2 mg/mL. The reaction system underwent a reaction for 16 h at 16 °C. The obtained reaction system was held for 5 min at 80 °C to deactivate the ligase, a sediment was removed through centrifugation at 12000 rpm, and supernatant was detected through UPLC. Results were shown in Table 7. Results showed that the generation of products could be detected.

**Table 6**

| Substrate | Sequence (5'-3') | 5' end | 3' end |
|---|---|---|---|
| Substrate 11 (SEQ ID NO: 53) | Cms-Ums-Am-Gm-Am-Cm-Cf-Um | Hydroxyl group | Hydroxyl group |
| Substrate 12 (SEQ ID NO: 54) | Gf-Um-dT-Um-Um-Gm-Cm-Um-Um-Um- Um-Gm-Um | Phosphate group | L96 |
| Substrate 13 (SEQ ID | Am-Af-Am-Af-Cm-Af-Gm-Gf-Um-Cf-Um- | Phosphate | Hydroxyl |
| NO: 55) | Am-Gms-Ams-Am | group | group |
| Substrate 14 (SEQ ID NO: 56) | Ams-Cfs-Am-Af-Af-Af-Gm-Cf | Hydroxyl group | Hydroxyl group |

The m after A, C, G, or U represented 2' methoxy modification for the ribonucleotide, the f represented 2' fluoro modification for the ribonucleotide, the s represented the phosphorothioate bond, the d before A, C, G, or T represented that the nucleotide was deoxyribonucleotide, and L96 represented the N-acetylgalactosamine group derivative.

The ribonucleotides at positions 1, 2, 3, 4, 5, 6, and 8 of the substrate 11 had 2' methoxy modifications; the ribonucleotide at position 7 had 2' fluoro modification; and the ribonucleotides at positions 1 and 2 were ligated by a phosphorothioate bond, and the ribonucleotides at positions 2 and 3 were ligated by a phosphorothioate bond.

The ribonucleotides at positions 2, 4, 5, 6, 7, 8, 9, 10, 11, 12, and 13 of the substrate 12 had 2' methoxy modifications; the ribonucleotide at position 1 had 2' fluoro modification; and the ribonucleotide at position 3 was the deoxyribonucleotide, which was the thymine (T).

The ribonucleotides at positions 1, 3, 5, 7, 9, 11, 12, 13, 14, and 15 of the substrate 13 had 2' methoxy modifications; the ribonucleotides at positions 2, 4, 5, 6, 8, and 10 had 2' fluoro modifications; and the ribonucleotides at positions 13 and 14 were ligated by a phosphorothioate bond, and the ribonucleotides at positions 14 and 15 were ligated by a phosphorothioate bond.

The ribonucleotides at positions 1, 3, and 7 of the substrate 14 had 2' methoxy modifications; the ribonucleotides at positions 2, 4, 5, 6, and 8 had 2' fluoro modifications; and the ribonucleotides at positions 1 and 2 were ligated by a phosphorothioate bond, and the ribonucleotides at positions 2 and 3 were ligated by a phosphorothioate bond.

**Table 7**

| Enzyme | Activity | |
|---|---|---|
| | Sense strand product | Antisense strand product |
| E1 | × | × |
| E2 | × | × |
| E3 | × | × |
| E4 | + | + |
| E5 | + | + |
| E6 | +++ | +++ |
| E7 | +++ | +++ |
| E8 | ++ | ++ |
| E9 | +++ | +++ |
| E10 | +++ | +++ |
| E11 | × | × |
| E12 | × | × |
| E13 | +++ | +++ |
| E14 | +++ | +++ |
| E15 | × | - |
| E16 | ++ | ++ |
| E17 | +++ | +++ |
| E18 | +++ | +++ |
| E19 | +++ | +++ |
| E20 | +++ | +++ |
| E21 | +++ | +++ |
| E22 | +++ | +++ |
| E23 | +++ | +++ |
| E24 | +++ | +++ |
| E25 | +++ | +++ |
| E26 | +++ | +++ |
| E27 | +++ | +++ |
| E28 | +++ | +++ |
| E29 | +++ | +++ |
| E30 | +++ | +++ |
| E31 | +++ | ++ |
| E32 | ++ | +++ |
| E33 | +++ | +++ |
| E34 | +++ | +++ |
| E35 | +++ | +++ |
| E36 | +++ | ++ |
| E37 | +++ | +++ |
| E38 | +++ | +++ |
| E39 | ++ | ++ |
| E40 | +++ | ++ |

In the indication of activity, "-" indicated that the purity of the product system was 0.1-5% (excluding an endpoint value of 5%); "+" indicated that the purity of the product system was 5%-20% (excluding an endpoint value of 20%); "++" indicated that the purity of the product system was 20%-30% (excluding an endpoint value of 30%); "+++" indicated that the purity of the product system was 30%-50%; and "×" indicated that no product was produced.

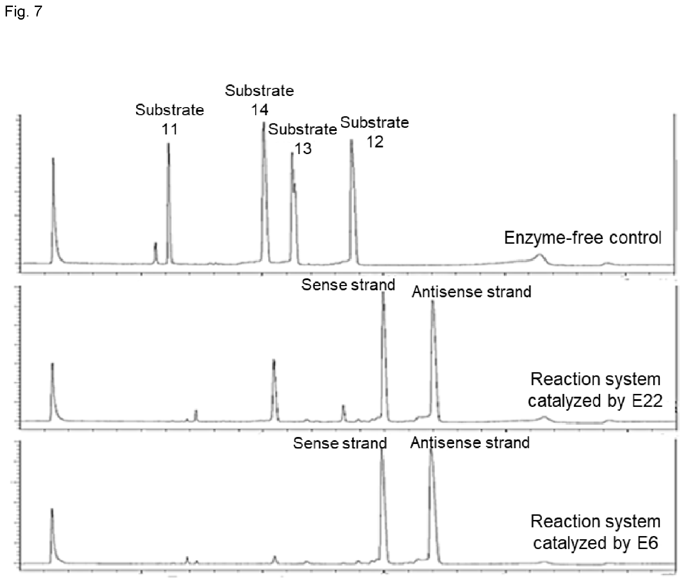

Fig. 7 showed a UPLC detection diagram of an enzyme-free control system and a reaction system catalyzed by partial enzymes.

### Comparative example 1

The target siRNA was synthesized by the solid-phase synthesis method (solid-phase synthesizer) in the prior art, its product purity was determined, and impurity profiling was performed by mass spectrometry. By comparing the target siRNA synthesized by the solid-phase method with the same concentration of target siRNA synthesized enzymatically using the E - 7 enzyme, it was found that the enzymatically synthesized product exhibited higher purity.. Purity data was shown in Table 8.

**Table 8**

| Sample type | Non-denaturation purity | Denaturation purity |
|---|---|---|
| siRNA prepared through enzymatic method | 95.00% | 96.30% |
| siRNA prepared through solid-phase synthesis method | 94.90% | 94.60% |

Further, the content of the impurities was detected using UPLC mass spectrometry. Results showed that the N-1 and N+1 impurities in the enzymatically-synthesized target siRNA were significantly lower than those in the target siRNA synthesized by the solid-phase synthesis method. Results for impurity content were shown in Table 9. It indicated that the target siRNA synthesized through the enzymatic method was more advantageous.

**Table 9**

| Sample | Chain | Mass spectrometry data analysis | | UPLC data | |
|---|---|---|---|---|---|
| | | N-1% | N+1% | N-1% | N+1% |
| SS sample obtained through solid-phase synthesis | SS | 1.13% | 0.054% | 1.30% | ND |
| AS sample obtained through solid-phase synthesis | AS | 1.80% | 0.18% | 1.25% | ND |
| Sample obtained through enzyme-catalyzed synthesis | SS | 0.30% | 0.035% | 0.22% | ND |
| | AS | 0.30% | 0.055% | 0.36% | ND |

The ND indicated not detected.

It may be seen from the above description that, in the above embodiments of the present disclosure, the following technical effects are realized. In the method of the present application, the ligation between the sense strand substrates is catalyzed by the RNA ligase, so as to form the sense strand of the target siRNA, and the ligation between the antisense strand substrates is catalyzed to form the antisense strand of the target siRNA, such that the siRNA with a complex structure and a plurality of modifications is prepared by means of biosynthesis. Compared to the preparation of the target siRNA using chemical synthesis, the method of the present application is high in purity of products obtained, less in impurity, small in purification pressure of the final product, mild in reaction condition, and free of use of a large number of organic reagents, such that production costs can be reduced, and industrial scale-up production is achieved.

The above are only the preferred embodiments of the present disclosure and are not intended to limit the present disclosure. For person skilled in the art, the present disclosure may have various modifications and variations. Any modifications, equivalent replacements, improvements and the like made within the spirit and principle of the present disclosure all fall within the scope of protection of the present disclosure.

## Claims

1. A method for preparing a siRNA that inhibits the expression of a PCSK9 gene, wherein the siRNA is a double stranded RNA consisting of a sense strand and an antisense strand which are complementary paired;
the method comprises:
mixing a sense strand substrate, an antisense strand substrate, and an RNA ligase, and utilizing the RNA ligase to catalyze the ligation between the sense strand substrate and the ligation between the antisense strand substrate with phosphodiester bonds to obtain the sense strand and the antisense strand, thereby obtaining the siRNA;
wherein the sense strand substrate is capable of constituting the sense strand, and the antisense strand substrate is capable of constituting the antisense strand; and
the sense strand is a nucleic acid as set forth in SEQ ID NO: 45, and the antisense strand is a nucleic acid as set forth in SEQ ID NO: 46.

2. The method according to claim 1, wherein the 5' end of the sense strand is a hydroxyl group, and the 3' end of the sense strand is an L96 group, the ribonucleotides at positions 1, 2, 3, 4, 5, 6, 8, 10, 12, 13, 14, 15, 16, 17, 18, 19, 20, and 21 of the sense strand have 2' methoxy modifications; the ribonucleotides at positions 7 and 9 have 2' fluoro modifications; the ribonucleotides at positions 1 and 2 are ligated by a phosphorothioate bond, the ribonucleotides at positions 2 and 3 are ligated by a phosphorothioate bond; and the position 11 of sense strand is deoxyribonucleotide, which is a thymine;
the 5' end of the antisense strand is a hydroxyl group, and the 3' end of the antisense strand is a hydroxyl group, the ribonucleotides at positions 1, 3, 7, 9, 11, 13, 15, 17, 19, 20, 21, 22 and 23 of the antisense strand have 2' methoxy modifications; the ribonucleotides at positions 2, 4, 5, 6, 8, 10, 12, 14, 16 and 18 have 2' fluoro modifications, the ribonucleotides at positions 1 and 2 are ligated by a phosphorothioate bond, the ribonucleotides at positions 2 and 3 are ligated by a phosphorothioate bond, the ribonucleotides at positions 21 and 22 are ligated by a phosphorothioate bond, and the ribonucleotides at positions 22 and 23 are ligated by a phosphorothioate bond.

3. The method according to claim 1, wherein the RNA ligase comprises one or more of the RNA ligases as shown in SEQ ID NO: 4 to SEQ ID NO: 10 or SEQ ID NO: 13 to SEQ ID NO: 40; or
an enzyme having greater than 70% identity to any one of the RNA ligases as shown in SEQ ID NO: 4 to SEQ ID NO: 10 or SEQ ID NO: 13 to SEQ ID NO: 40 and having an activity of catalyzing the formation of the phosphodiester bond.

4. The method according to any one of claims 1 to 3, wherein the sense strand substrate comprises 2 or more substrates, and the antisense strand substrate comprises 2 or more substrates.

5. The method according to claim 4, wherein the sense strand substrate has a length of 2-19 nt, and the antisense strand substrate has a length of 2-21 nt.

6. The method according to claim 4, wherein both the sense strand substrate and the antisense strand substrate comprise two substrates, wherein the sense strand substrate comprises a first sense strand substrate and a second sense strand substrate, and the antisense strand substrate comprises a first antisense strand substrate and a second antisense strand substrate;
the method comprises: mixing the first sense strand substrate, the second sense strand substrate, the first antisense strand substrate, and the second antisense strand substrate, utilizing the RNA ligase to catalyze the ligation between the first sense strand substrate and the second sense strand substrate to form the sense strand, and to catalyze the ligation between the first antisense strand substrate and the second antisense strand substrate to form the antisense strand, and the sense strand and the antisense strand are complementary paired to form the siRNA.

7. The method according to claim 6, wherein the sense strand substrate and the antisense strand substrate are annealed, and then mixed with the RNA ligase, so as to obtain the siRNA.

8. The method according to claim 6, wherein the 3' end of the first sense strand substrate is ligated to the 5' end of the second sense strand substrate under the catalysis of the RNA ligase, forming the sense strand; and the 3' end of the first antisense strand substrate is ligated to the 5' end of the second antisense strand substrate under the catalysis of the RNA ligase, forming the antisense strand.

9. The method according to claim 8, wherein the 5' end of the first sense strand substrate is a hydroxyl group, and the 3' end of the first sense strand substrate is a hydroxyl group; and the 5' end of the second sense strand substrate is a phosphate group, and the 3' end of the second sense strand substrate is an L96 group; and
the 5' end of the first antisense strand substrate is a hydroxyl group, and the 3' end of the first antisense strand substrate is a hydroxyl group; and the 5' end of the second antisense strand substrate is a phosphate group, and the 3' end of the second antisense strand substrate is a hydroxyl group.

10. The method according to claim 8 or 9, wherein the first sense strand substrate is a nucleic acid as set forth in SEQ ID NO: 41; and the second sense strand substrate is a nucleic acid as set forth in SEQ ID NO: 42; or
the first sense strand substrate is a nucleic acid as set forth in SEQ ID NO: 53, and the second sense strand substrate is a nucleic acid as set forth in SEQ ID NO: 54.

11. The method according to claim 8 or 9, wherein the first antisense strand substrate is a nucleic acid as set forth in SEQ ID NO: 44, and the second antisense strand substrate is a nucleic acid as set forth in SEQ ID NO: 43; or
the first antisense strand substrate is a nucleic acid as set forth in SEQ ID NO: 56, and the second antisense strand substrate is a nucleic acid as set forth in SEQ ID NO: 55.

12. The method according to claim 4, wherein both the sense strand substrate and the antisense strand substrate comprise 3 substrates,
the sense strand substrate comprises a first sense strand substrate, a second sense strand substrate, and a third sense strand substrate;
the antisense strand substrate comprises a first antisense strand substrate, a second antisense strand substrate, and a third antisense strand substrate.

13. The method according to claim 12, wherein the first sense strand substrate is a nucleic acid as set forth in SEQ ID NO: 47;
the second sense strand substrate is a nucleic acid as set forth in SEQ ID NO: 48; and
the third sense strand substrate is a nucleic acid as set forth in SEQ ID NO: 49.

14. The method according to claim 12, wherein the first antisense strand substrate is a nucleic acid as set forth in SEQ ID NO: 52;
the second antisense strand substrate is a nucleic acid as set forth in SEQ ID NO: 51; and
the third antisense strand substrate is a nucleic acid as set forth in SEQ ID NO: 50.

15. The method according to any one of claims 1 to 3, wherein the concentrations of the sense strand substrate and the antisense strand substrate are both 1 µM to 1M; and
the concentration of the RNA ligase is 0.01 mg/mL to 10 mg/mL.

16. The method according to claim 15, wherein in the a reaction system formed by mixing the sense strand substrate, the antisense strand substrate and the RNA ligase, ATP, Tris-HCl, MgCl₂, and DTT are further comprised.

17. The method according to claim 15, wherein the reaction temperature of the method is 0-40°C, and
the reaction time of the method is 0.1-48 hours.
